(19) Europäisches Patentamt
European Patent Office
Office européen des brevets

(11) Numéro de publication : **0 383 686 B1**

# (12) FASCICULE DE BREVET EUROPEEN

(45) Date de publication du fascicule du brevet : **04.08.93 Bulletin 93/31**

(51) Int. Cl.$^5$ : **C07C 235/36,** A61K 31/165, C07C 217/74

(21) Numéro de dépôt : **90400405.8**

(22) Date de dépôt : **14.02.90**

## (54) Carboxyalkyl-ethers de la 2-amino-7-hydroxytetraline.

(30) Priorité : **14.02.89 FR 8901910**

(43) Date de publication de la demande : **22.08.90 Bulletin 90/34**

(45) Mention de la délivrance du brevet : **04.08.93 Bulletin 93/31**

(84) Etats contractants désignés : **AT BE CH DE DK ES FR GB GR IT LI LU NL SE**

(56) Documents cités :
**EP-A- 0 211 721**
**EP-A- 0 253 257**

(73) Titulaire : **ELF SANOFI**
**32-34, rue Marbeuf**
**F-75008 Paris (FR)**
(84) **BE CH DE DK ES FR GB GR LI LU NL SE AT**
Titulaire : **MIDY S.p.A.**
**Via Piranesi 38**
**I-20137 Milano (IT)**
(84) **IT**

(72) Inventeur : **Guzzi, Umberto**
**Via Don Gnocchi 28**
**I-20148 Milano (IT)**
Inventeur : **Cecchi, Roberto**
**Via Dei Tigli 1**
**I-20075 Lodi Milan (IT)**

(74) Mandataire : **Gillard, Marie-Louise et al**
**Cabinet Beau de Loménie 158, rue de l'Université**
**F-75340 Paris Cédex 07 (FR)**

**EP 0 383 686 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des carboxyalkyl-éthers de la 2-amino-7-hydroxytétraline, un procédé et les intermédiaires pour leur préparation, leur utilisation comme produits de départ dans la préparation de composés pharmacologiquement actifs et les nouvelles phényléthanolaminotétralines 7-substituées ainsi obtenues ayant une activité spasmolytique.

Plus particulièrement, la présente invention a pour objet, selon un de ses aspects, des carboxyalkyl-éthers de la 2-amino-7-hydroxytétraline de formule

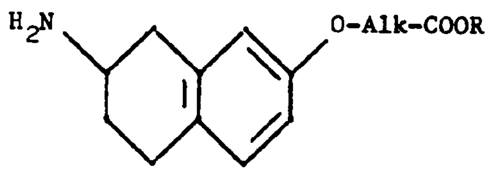

(I)

dans laquelle

- Alk représente un groupe alkylène de 3 à 5 atomes de carbone, à chaîne droite ou ramifiée, et
- R représente l'hydrogène ou un groupe alkyle de 1 à 4 atomes de carbone,

et leurs sels.

En particulier, le terme Alk peut représenter

(a) un groupe

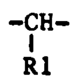 , 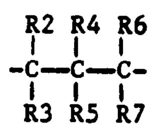 , 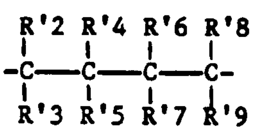 ,

ou $-(CH_2)_5-$

où

R1 est un groupe éthyle, propyle ou butyle ;

R2 à R7 sont tous des hydrogènes ou un des radicaux R2 à R7 est un groupe méthyle ou éthyle et les autres sont des hydrogènes ou deux des radicaux R2 à R7 sont des groupes méthyle et les autres sont des hydrogènes ;

R'2 à R'9 sont tous des hydrogènes ou un des radicaux R'2 à R'9 est un groupe méthyle et les autres sont des hydrogènes.

(b) un groupe

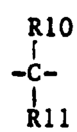

où

R10 et R11 représentent indépendamment un groupe méthyle ou éthyle, ou

R11 est aussi le groupe propyle lorsque R10 est le groupe méthyle ;

(c) un groupe

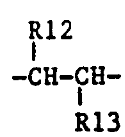

où

l'un des radicaux R12 et R13 est l'hydrogène et l'autre est le groupe méthyle, éthyle ou propyle, ou l'un

des radicaux R12 et R13 est le groupe méthyle et l'autre est le groupe méthyle ou éthyle.

Les nouveaux composés sont utiles comme produits de départ dans la préparation de composés pharmaceutiquement actifs, en particulier de phényléthanolaminotétralines 7-substituées ayant une activité spasmolytique.

Dans la présente description, le terme "tétraline" se rapporte au 1,2,3,4-tétrahydronaphtalène, et le terme "2-tétralone" au dérivé 2-oxo correspondant.

Les composés de formule (I) et leur sels peuvent être préparés selon la méthode suivante, qui représente un autre objet de la présente invention et qui est caractérisée en ce que:

(A) on soumet une 2-amino-7-hydroxytétraline N-protégée de formule (II)

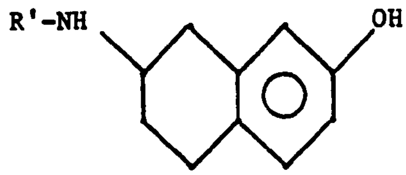

(II)

dans laquelle R' est un groupe N-protecteur susceptible d'être éliminé par hydrogénation catalytique ou par hydrolyse acide douce, à une réaction de carb(alk)oxyalkylation avec un composé de formule (IIIa)

$$Hal - Alk^1 - COOR \quad (IIIa)$$

dans laquelle R est tel que défini ci-dessus, Hal représente un atome de chlore, brome ou iode et $Alk^1$ représente un groupe alkylène tel que défini ci-dessus au point (a), en présence d'un agent de condensation basique,

ou avec un compose de formule (IIIb)

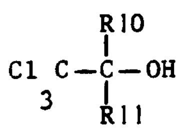

(IIIb),

dans laquelle R10 et R11 sont tels que définis ci-dessus au point (b), en présence d'une base forte, suivie éventuellement d'une réaction du produit ainsi obtenu avec du chlorure de thionyle dans un alcanol en $C_1$-$C_4$ convenablement choisi ;

ou avec un composé de formule (IIIc)

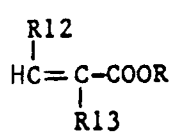

(IIIc)

dans laquelle R est tel que défini ci-dessus, et R12 et R13 sont tels que définis ci-dessus au point (c), en présence éventuellement d'une quantité catalytique d'un hydroxyde d'ammonium quaternaire,

pour obtenir un carboxyalkyl-éther d'une 2-amino-7-hydroxytétraline N-protégée de formule (IV)

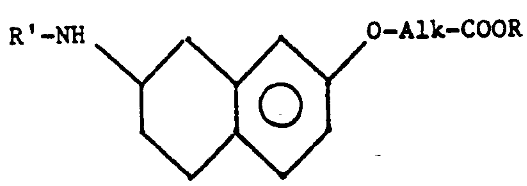

(IV)

dans laquelle Alk, R et R' sont tels que définis ci-dessus, et

(B) on élimine le groupe N-protecteur par hydrogénation catalytique ou par hydrolyse acide douce et, après avoir éventuellement saponifié le groupe carbalkoxy inférieur en groupe carboxy, on isole le composé de formule (I), sous forme de base libre ou de sel, et on le transforme éventuellement en l'un de ses sels.

Le terme "carb(alk)oxyalkylation" désigne en général la réaction de condensation de l'hydroxyle phénolique avec un réactif capable d'éthérifier ledit hydroxyle avec un groupe alkyle substitué par un groupe carb(alk)oxy, par exemple carboxy ou carbalcoxy, le terme "carbalcoxy" désignant un groupe ($C_1$-$C_4$ alcoxy)carbonyle.

Les groupes N-protecteurs préférés sont les groupes tert-butoxycarbonyle (Boc), benzyloxycarbonyle et, en général, les groupes N-protecteurs utilisés dans la chimie des peptides, ou les groupes benzyle, benzhydryle ou trityle, non substitués ou substitués par un groupe méthoxy dans un cycle benzènique.

Comme composé de formule (IIIa), on utilise de préférence un bromo-alcanoate d'alkyle. La réaction entre la 2-amino-7-hydroxytétraline N-protégée (II) et le composé (IIIa) est conduite dans un solvant organique tel que l'acétone, l'acétate d'éthyle ou le tétrahydrofuranne en utilisant un agent de condensation basique classique comme un carbonate alcalin, par exemple le carbonate potassique.

Le composé de formule IIIb est utilisé, comme décrit dans J. Am. Chem. Soc., 1948, 70, 1153-1158, lorsqu'on veut préparer un composé de formule I où Alk est un groupe -C(R10R11)-. Dans ce cas, la réaction est conduite de préférence en présence d'une base forte, telle que l'hydroxyde de sodium ou de potassium et, éventuellement, on obtient le produit I sous forme d'ester par traitement de l'acide ainsi obtenu avec du chlorure de thionyle dans un alcanol convenablement choisi.

La réaction entre la 2-amino-7-hydroxytétraline N-protégée (II) et le dérivé de l'acide acrylique de formule (IIIc) peut être conduite aussi bien en l'absence qu'en présence d'un solvant organique, inerte, apolaire tel que le benzène, le toluène, l'éther éthylique, le chlorure de méthylène. La réaction peut être catalysée, si on le désire, par le petites quantités d'un hydroxyde d'ammonium quaternaire, par exemple l'hydroxyde de benzyltriméthylammonium. De préférence, pour éviter l'addition de l'acrylate à la liaison entre le groupe amino et la tétraline, lorsqu'on utilise un composé de formule (IIIc), le groupe amino du partenaire de réaction (II) est protégé par le groupe Boc ou par l'une quelconque des groupes du type uréthane aisement éliminables.

Le carboxyalkyl-éther de la 2-amino-7-hydroxytétraline N-protégée (IV) ainsi obtenu est isolé selon les techniques conventionnelles, éventuellement sous forme d'un de ses sels, et soumis à la N-déprotection.

L'élimination des groupes N-protecteurs est effectuée par hydrogénation catalytique ou par hydrolyse acide douce selon des méthodes bien connues de littérature. Notamment, le groupe Boc est éliminé dans des conditions acides, par action de l'acide trifluoroacétique. Les autres groups énumérés ci-dessus sont tous éliminés par hydrogénation catalytique, de préférence en utilisant le palladium sur charbon comme catalyseur. Les groupes trityle et méthoxytrityle peuvent également être hydrolysés dans des conditions acides douces, par exemple par l'acide formique à 50% ou par l'acide chlorhydrique gazeux dans un solvant organique.

Les composés de formule I peuvent être soumis à une saponification pour obtenir le groupe carboxy correspondant, avant ou après la déprotection du groupe amino.

Les produits I sont isolés selon les méthodes conventionnelles, de préférence sous forme d'un de leurs sels d'addition avec les acides minéraux ou organiques qui permettent une séparation ou une cristallisation convenable des composés, tels que l'acide picrique, l'acide oxalique ou un acide optiquement actif, par exemple un acide mandélique ou un acide camphosulfonique, ou avec les acides minéraux ou organiques qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalènesulfonate.

La base peut être libérée par neutralisation et transformée dans un autre sel d'addition avec des acides ou, lorsque R est l'hydrogène, en un sel avec un métal, notamment alcalin, tel que le sel de sodium, selon des procédures conventionnelles.

La 2-amino-7-hydroxytétraline N-protégée de formule (II) est préparée soit à partir de la 2-amino-7-hydroxytétraline de formule (IIa)

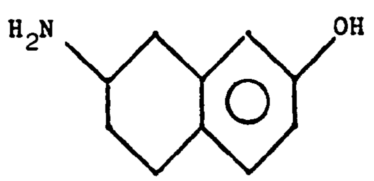

**(IIa)**

soit directement à partir de la 7-méthoxy-2-tétralone de formule (V)

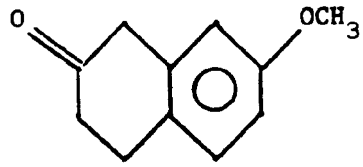

(V)

sans passer par la 2-amino-7-hydroxytétraline IIa.

La 2-amino-7-hydroxytétraline (IIa) peut être préparée à partir de la méthoxytétralone correspondante de formule V par réaction avec la benzylamine, réduction de la benzylimine obtenue par du borohydrure de sodium, débenzylation par hydrogénation catalytique et déméthylation par l'acide bromhydrique à 48%, selon le Schéma 1 suivant

**SCHEMA 1**

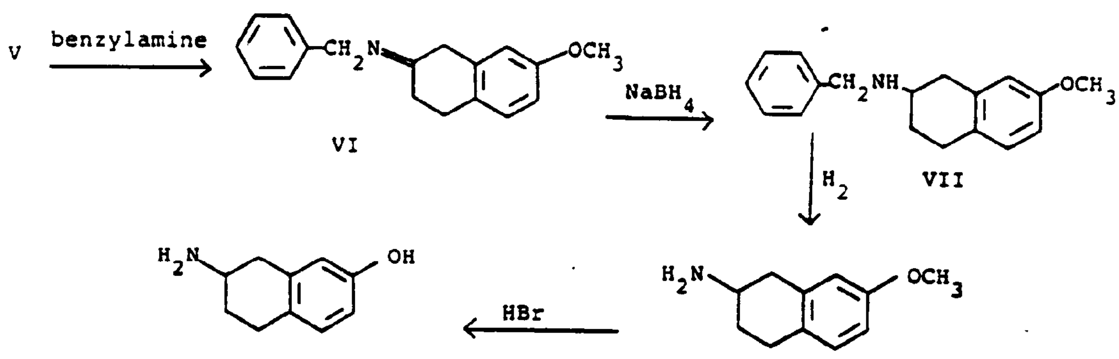


V, VI, VII, VIIa, IIa

La réaction du produit V avec la benzylamine est effectuée selon les méthodes classiques de préparation des bases de Schiff, dans un solvant organique tel que le toluène, en présence d'acide p-toluène-sulfonique et le composé VI ainsi obtenu peut être réduit, sans être isolé et purifié, avec le borohydrure de sodium. Par hydrogénation catalytique, en utilisant par exemple du palladium sur charbon, on obtient la 2-amino-7-méthoxytétraline (VIIa) qui, par chauffage dans de l'acide bromhydrique à 48%, donne la 2-amino-7-hydroxytétraline (IIa), comme bromhydrate, transformé en la base libre par neutralisation.

Dans la première étape du Schéma 1 ci-dessus, la benzylamine peut être remplacée par la tritylamine ou la benzhydrylamine, les trois produits pouvant être substitués par un groupe méthoxy sur un noyau phényle.

Les composés de formule (II), (IIa), (VII) et (VIIa) ont un centre d'asymétrie sur l'atome de carbone lié au groupe amino. La préparation de sels d'addition de ces composés avec un acide organique chiral, de préférence un acide mandélique optiquement actif, suivie par une cristallisation fractionnée, peut aboutir à des composés qui sont enrichis en l'un des deux énantiomères et, ensuite au dédoublement des racémiques en obtenant les deux formes optiquement actives. Le dédoublement de ces composés peut être aussi achevé par des techniques chromatographiques particulières.

La N-protection par le groupe R' est effectuée en faisant réagir le composé de formule (IIa) avec le réactif approprié pour la protection des groupes amino comme décrit, par exemple, par M. Bodanszky et al., Peptide Synthesis, 2nd Edition, John Wiley & Sons 1976, pages 18 à 49, chapitres 3 à 6.

Le groupe Boc, par exemple, peut être introduit par réaction avec le di-tert-butyldicarbonate en milieu basique. Le groupe benzyloxycarbonyle peut être introduit selon la procédure générale décrite par E.C. Horning, Organic Synthesis, vol. III, Wiley, New York 1955, page 167.

Ainsi, par exemple, pour préparer les produits (I), on peut protéger le groupe amino de la 2-amino-7-hydroxytétraline (IIa) par un groupe Boc par réaction de l'aminotétraline (IIa) avec le di-tert-butyl-dicarbonate dans un solvant organique tel que le dioxane ou le diméthylformamide, traiter le produit ainsi obtenu avec un composé de formule (IIIa) ou (IIIb) ou (IIIc) dans les conditions ci-dessus et déprotéger le groupe amino par élimination du groupe Boc avec de l'acide trifluoroacétique selon le Schéma 2 suivant

SCHEMA 2

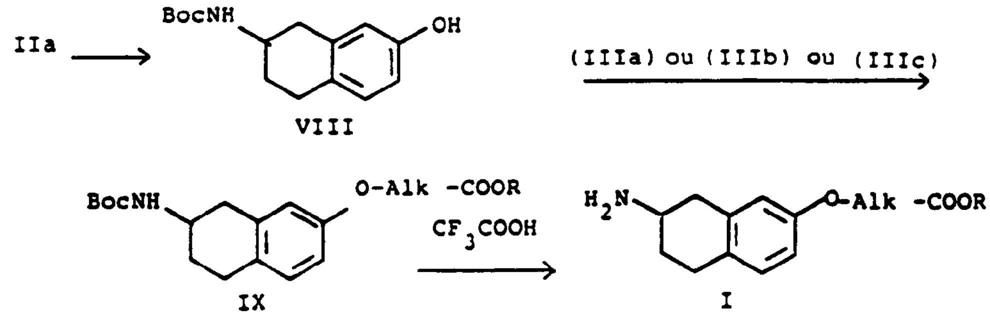


La libération de l'amine (I) par élimination du groupe N-protecteur Boc ne comporte pas de modification au niveau du groupe carbalcoxy inférieur ou de la stéréoconfiguration.

La N-protection peut également être effectuée à partir de la 7-méthoxy-2-tétralone (V), par formation d'une base de Schiff avec une amine choisie parmi la benzylamine, la benzhydrylamine et la tritylamine, non substituées ou substituées par un groupe méthoxy sur un noyau phényle et réduction par le borohydrure de sodium.

Ainsi, par exemple, les produits de formule (I) dans laquelle Alk représente un groupe alkylène tel que défini aux points (a) ou (b), peuvent convenablement être préparés à partir d'une 2-benzylamino-7-méthoxytétraline de formule (VII) (Schéma 1), par déméthylation avec de l'acide bromhydrique, réaction du phénol correspondant avec un composé de formule (IIIa) ou (IIIb) dans les conditions ci-dessus et débenzylation, selon le Schéma 3 suivant

SCHEMA 3

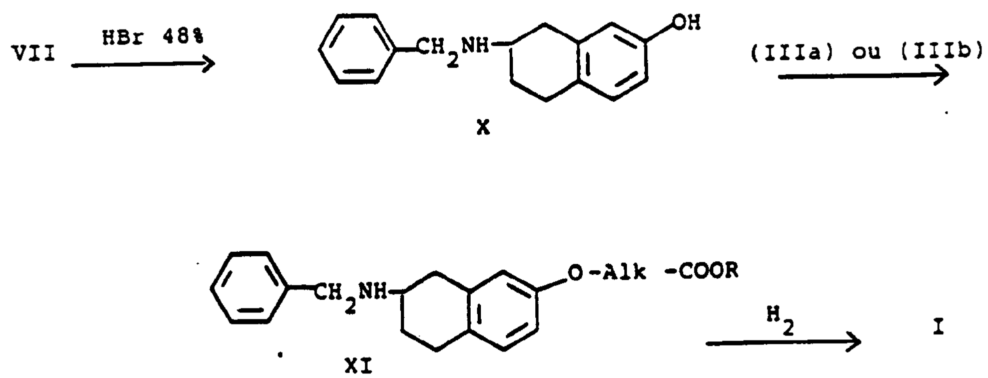


Dans le Schéma 2 ci-dessus, le groupe Boc peut être remplacé par le groupe benzyloxycarbonyle ou par tout autre groupe protecteur tel que défini ci-dessus. Dans le Schéma 3 le groupe benzyle peut être substitué sur le cycle benzénique par un groupe méthoxy ou peut être remplacé par un groupe benzhydryle ou trityle non substitué ou substitué sur le cycle benzénique par un groupe méthoxy. La déprotection est effectuée comme indiqué ci-dessus.

Les formes optiquement actives des produits de formule (I) sont préparées selon des méthodes connues, soit à partir du composé (IIa) optiquement actif, selon le Schéma 2, soit, selon le Schéma 3, après résolution du composé (VII) ou de son analogue benzhydryle ou trityle, éventuellement méthoxylé, soit par résolution du même composé (I), ladite résolution étant effectuée, par exemple, par salification avec un acide optiquement actif, l'acide mandélique optiquement actif de préférence.

Les carboxyalkyl-éthers de la 2-amino-7-hydroxytétraline N-protégée (IV) et leurs sels sont des produits nouveaux et représentent les intermédiaires clés dans la préparation des composés (I); ces produits (IV), sous forme racémique ou sous forme de leurs stéréoisomères séparés, représentent un autre aspect de la présente invention.

Les carboxyalkyl-éthers de la 2-amino-7-hydroxytétraline de formule (I), et leurs sels, sont des produits

utiles comme intermédiaires dans la préparation de composés pharmacologiquement actifs. Par exemple, ils peuvent être utilisés pour la préparation de phényléthanolaminotétralines ayant une activité agoniste des récepteurs béta-adrénergiques sélective pour le tractus gastro-intestinal et destinées à la préparation de médicaments spasmolytiques.

Ainsi, selon un aspect ultérieur, la présente invention a pour objet l'utilisation des carboxyalkyl-éthers de la 2-amino-7-hydroxytétraline de formule (I) pour la préparation de phényléthanolaminotétralines correspondantes de formule (XII):

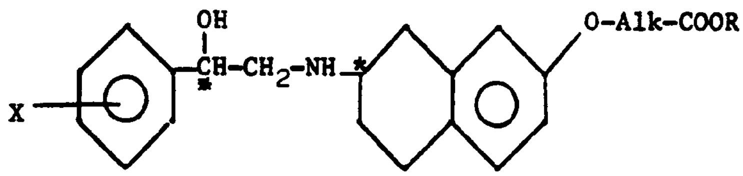

(XII)

dans laquelle X représente l'hydrogène, un halogène, un alkyle en $C_1$-$C_4$, ou un groupe trifluorométhyle, Alk et R sont tels que définis ci-dessus, et de leurs sels pharmaceutiquement acceptables.

Pour la préparation des composés (XII) on peut faire réagir les composés (I) soit avec un oxyde de styrène de formule (XIII)

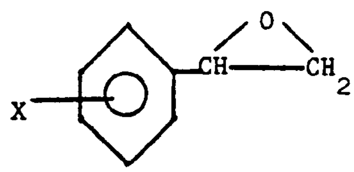

(XIII)

dans laquelle X est tel que défini ci-dessus, ledit époxyde de styrène pouvant être racémique ou optiquement actif,
soit avec un dérivé fonctionnel d'un acide mandélique de formule (XIV)

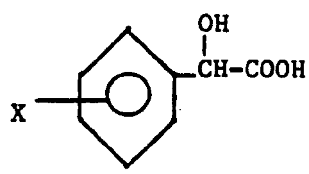

(XIV)

dans laquelle X est tel que défini ci-dessus et, ensuite, réduire le groupe carbonyle amidique du mandélamide ainsi obtenu de formule

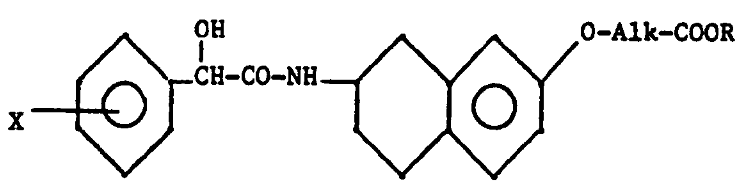

(XV)

dans laquelle X, Alk et R sont tels que définis ci-dessus, en groupe méthylène, ledit acide mandélique (XIV) pouvant être racémique ou optiquement actif;
et éventuellement transformer les produits ainsi obtenus en leurs sels pharmaceutiquement acceptables.

La réaction entre les composés de formule (I) et les oxydes de styrène (XIII) peut être conduite en l'absence de solvants ou en présence d'un solvant organique, inerte, de préférence polaire, et éventuellement en présence d'une quantité équimolaire de N-triméthylsilylacétamide pour contrôler l'ouverture de l'époxyde.

La réaction, qui est complète, en général, après quelques heures, normalement après 6 à 24 heures, peut être conduite à la température ambiante ou, de préférence, à une température supérieure, généralement de 50 à 90°C, pour accélerer le cours de la réaction.

Comme "dérivé fonctionnel" de l'acide mandélique (XIV), on peut utiliser le chlorure, l'anhydride, une anhydride mixte, un ester actif ou l'acide libre convenablement activé, par exemple, avec le dicyclohexylcarbodiimide ou avec l'hexafluorophosphate de benzotriazolyl-N-oxytris-(diméthylamino)phosphonium (BOP). De préférence, on utilise un acide mandélique activé avec un agent de condensation tel que le BOP.

La réaction du dérivé fonctionnel de l'acide mandélique (XIV) avec un composé de formule (I) est en général conduite dans un solvant organique inerte tel que le chlorure de méthylène, éventuellement en présence d'un accepteur de protons, tel que la triéthylamine.

Le mandélamide (XV) ainsi obtenu est alors soumis à la réduction du groupe amido en groupe méthylèneamino en donnant le produit désiré de formule (XII), par action du diborane ou d'un réactif générant le diborane tel que le complexe entre le borane et le diméthylsulfure, communément désigné "borane-méthylsulfure". La réaction est conduite dans un solvant organique tel que le tétrahydrofuranne.

Indépendamment de la méthode de synthèse, le composé désiré de formule (XII) est isolé et purifié selon les techniques connues.

Les composés de formule (XII) présentent deux centres d'asymétrie sur les atomes de carbone marqués par les astérisques dans la formule ci-dessus. Dans la préparation de ces composés, en partant d'un des énantiomères (I) et d'un des énantiomères (XIII) ou (XIV), on obtient les stéréoisomères purs des composés (XII). La réaction en jeu est stéréoconservative et la même configuration absolue de deux atomes de carbone chiraux des substrats (I), (XIII) et (XIV), telle qu'assignée selon la Convention (R,S), est rétenue dans le produit final (XII).

En utilisant un des produits de départ sous forme de racémique, on obtient un mélange de deux diastéréoisomères alors qu'en utilisant les deux réactifs sous forme de racémiques on obtient un mélange de quatre stéréoisomères. La cristallisation fractionnée des diastéréoisomères ou la chromatographie du mélange peut aboutir à des composés qui sont enrichis en l'un des diastéréoisomères possibles ou même aux stéréoisomères purs.

Les énantiomères des composés de formule (XII) dans laquelle X, Alk et R ont la signification donnée ci-dessus, et leurs sels pharmaceutiquement acceptables, ainsi que les mélange d'énantiomères ou de diastéréoisomères en toutes proportions représentent donc un autre objet de la présente invention.

Un groupe préféré de composés de formule (XII) comprend les composés de formule (XII) dans laquelle X et R sont tels que définis ci-dessus et Alk représente un groupe -C(R10R11)- ou un groupe $Alk^1$ où R10, R11 et $Alk^1$ sont tels que définis ci-dessus, sous forme d'énantiomères purs ou de mélanges d'énantiomères ou de diastéréoisomères en toutes proportions, et leurs sels d'addition avec des acides pharmaceutiquement acceptables.

Un groupe encore plus préféré de composés de formule (XII) comprend les composés de formule (XII) dans laquelle X et R sont tels que définis ci-dessus et Alk représente un groupe -C(R10R11)- où R10 et R11 représentent un méthyle, sous forme d'énantiomères purs ou de mélanges d'énantiomères ou de diastéréoisomères en toutes proportions et leurs sels d'addition avec des acides pharmaceutiquement acceptables.

Les composés de formule (XII) se sont montrés plus actifs, en tant qu'agonistes des récepteurs bétaadrenergiques, et/ou plus sélectifs pour le tractus gastro-intestinal que le composé correspondant substitué en position 7 du noyau tétralinique par un groupe éthoxycarbonylméthoxy qui a été décrit dans le brevet européen EP-A-211721.

Les composés de formule (XII) selon la présente invention montrent une activité considérable sur la motricité intestinale et sont utiles en tant que spasmolytiques. La toxicité des composés (XII) et de leurs sels pharmaceutiquement acceptables est très faible et compatible avec leur utilisation en tant que principes actifs dans les compositions pharmaceutiques.

Selon la présente invention, les composés de formule (XII) peuvent être administrés à des doses de 0,01 à 10 mg par kg de poids du corps et par jour, selon la voie d'administration, le type du traitement, curatif ou prophylactique, l'âge du sujet à traiter et la gravité de la maladie.

Les composés de formule (XII) sont en général administrés en doses unitaires de 0,1 à 150 mg, de préférence de 1 à 50 mg, 1 à 5 fois par jour.

Ces doses unitaires sont de préférence formulées en compositions pharmaceutiques dans lesquelles le principe actif de formule (XII) est en combinaison avec un support pharmaceutique.

Un objet spécifique additionnel de la présente invention est donc une composition pharmaceutique utile pour le traitement des troubles gastrointestinaux associés à une contraction du muscle lisse, renfermant en tant que principe actif, un composé de formule (XII) ou un de ses sels pharmaceutiquement acceptables.

Les compositions pharmaceutiquement selon la présente invention peuvent être formulées pour l'administration orale, sublinguale, sous-cutanée, intramusculaire, intraveineuse, transdermique ou rectale.

Les compositions selon la présente invention peuvent être préparées selon des méthodes conventionnelles en utilisant des ingrédients et des excipients conventionnels comme il est connu dans le domain de la technique pharmaceutique.

Les exemples suivants illustrent l'invention sans toutefois la limiter.

PREPARATION I

Bromhydrate de 2-amino-7-hydroxytétraline.

(a) On chauffe au reflux pendant 3 heures un mélange de 8 g de 7-méthoxy-2-tétralone, 4,8 g de benzylamine, 150 ml de toluène anhydre et 100 mg d'acide p.toluènesulfonique. On évapore à sec l'huile résiduelle, on reprend par 100 ml de méthanol et à la solution obtenue on ajoute, avec précaution et à 0-5°C, 8,5 g de borohydrure de sodium. On laisse le mélange sous agitation et à la température ambiante pendant une nuit, puis on y ajoute 50 ml d'eau, on laisse sous agitation pendant 30 minutes, on évapore le solvant, on reprend avec 30 ml d'eau et 10 ml d'une solution concentrée d'hydroxyde d'ammonium. On extrait avec 200 ml d'acétate d'éthyle, on sèche la phase organique sur du sulfate de sodium, filtre et évapore à sec. On obtient une huile de couleur foncée qui est purifiée par flash chromatographie en utilisant comme éluant le mélange acétate d'éthyle/méthanol 95/5. On transforme la base obtenue en son chlorhydrate par dissolution dans 40 ml d'isopropanol et addition d'isopropanol saturé d'acide chlorhydrique gazeux. On obtient ainsi 11,4 g de chlorhydrate de 2-benzylamino-7-méthoxytétraline; p.f. 265-267°C (déc.).

(b) On soumet le produit ci-dessus, dissous dans 200 ml de méthanol et 100 ml d'eau, à une hydrogénation en présence de 1,2 g de palladium sur charbon à 10%, à la pression ambiante et à la température de 45-50°C. Après 4 heures on filtre, évapore à sec, reprend deux fois avec de l'éthanol absolu et on évapore à sec. On obtient un solide blanc qui est repris avec 70 ml d'isopropanol à chaud. Par refroidissement, la suspension obtenue précipite et donne 7,8 g de chlorhydrate de 2-amino-7-méthoxytétraline; p.f. 214-216°C.

(c) On met en suspension 6,6 g du produit ci-dessus dans 80 ml d'acide bromhydrique à 48% et on chauffe le mélange au reflux pendant 2 heures. On évapore à sec la solution obtenue, on reprend par de l'éthanol absolu et on évapore à sec deux fois. On obtient ainsi une huile qu'on dissout dans 20 ml d'isopropanol à chaud. Par addition de 30 ml d'éther éthylique à la solution on obtient 6,8 g de bromhydrate de 2-amino-7-hydroxytétraline cristallin; p.f. 171-173°C.

PREPARATION II

Monohydrate de R-2-amino-7-hydroxytétraline.

A une solution dans 550 ml d'éthanol absolu de 50 g de 2-amino-7-méthoxytétraline base brute, obtenue du chlorhydrate correspondant (PREPARATION I b) par neutralisation avec de l'hydroxyde de sodium à 10%, extraction avec de l'acétate d'éthyle et évaporation du solvant, on ajoute une solution de 43 g d'acide (+) mandélique dans 550 ml d'éthanol absolu. Après une nuit à la température ambiante, on filtre le précipité obtenu et on le cristallise deux fois dans l'éthanol absolu en récupérant chaque fois le produit cristallisé après repos d'une nuit à la température ambiante. On obtient ainsi 34,2 g (74%) de sel pur de l'acide (+) mandélique avec la (+)-2-amino-7-méthoxytétraline; p.f. 190-192°C. Les eaux mères de cette première cristallisation sont séparées et utilisées pour la PREPARATION III ci-dessous. On met en suspension 34 g du sel ainsi obtenu dans 300 ml d'eau et on rend basique le mélange réactionnel avec de l'hydroxyde de sodium N. On extrait la base avec de l'acétate d'éthyle, on évapore à sec et on reprend le résidu par 260 ml d'acide bromhydrique à 48%. On chauffe au reflux le mélange réactionnel pendant trois heures, on l'évapore à sec sous vide et on reprend le résidu obtenu avec 70 ml d'eau. On rend basique la solution aqueuse avec de l'hydroxyde d'ammonium concentré, on la refroidit pendant une nuit et on la filtre. On obtient 17 g de R-2-amino-7-hydroxytétraline sous forme monohydratée; p.f. 143-144°C, $[\text{alpha}]_D^{20}$ = + 85,1° (méthanol, c = 0,5 %).

Le chlorhydrate de ce produit a un pouvoir rotatoire qui correspond à celui de la littérature (Molecular Pharmacology, 1982, 22, 281-289).

PREPARATION III

Monohydrate de S-2-amino-7-hydroxytétraline.

On évapore à sec les eaux mères de la première cristallisation du produit de la PREPARATION II, contenant le sel de l'acide (+) mandélique avec la (-)-2-amino-7-méthoxytétraline, on suspend le résidu ainsi obtenu dans 300 ml d'eau et on rend la solution basique par de l'hydroxyde de sodium N. On extrait la base avec de l'acétate

d'éthyle. Suivant le mode opératoire décrit dans la PREPARATION II et en utilisant comme produits de départ la base ainsi obtenue et l'acide (-) mandélique, on obtient le sel de l'acide (-) mandélique avec la (-)-2-amino-7-méthoxytétraline (p.f. 189-191°C) qui, par neutralisation et déméthylation avec HBr, donne 17 g de S-2-amino-7-hydroxytétraline, sous forme de monohydrate; p.f. 143-144°C, $[alpha]_D^{20}$ = -86,9° (méthanol, c = 0,5 %). Le chlorhydrate de ce produit a un pouvoir rotatoire qui correspond à celui de la littérature (Molecular Pharmacology 1982, 22, 281-289).

PREPARATION IV

2-benzylamino-7-hydroxytétraline.

On chauffe au reflux pendant 2 heures, sous agitation, 25 g de chlorhydrate de 2-benzylamino-7-méthoxytétraline, PREPARATION I (a), dans 215 ml d'une solution à 33% d'acide bromhydrique dans l'acide acétique et en présence de 36 ml d'acide bromhydrique à 48%. Après concentration sous pression réduite, on reprend 3 fois le résidu avec 100 ml d'éthanol absolu en séchant chaque fois. On triture le produit ainsi obtenu avec 150 ml d'acétone, on filtre et on obtient 25,3 g du bromhydrate de 2-benzylamino-7-hydroxytétraline; p.f. 198-200°C. On dissout dans 1300 ml d'eau chaude le sel et, après refroidissement, on ajoute de l'hydroxyde d'ammonium concentré. On extrait la base par l'acétate d'éthyle, on sèche et on obtient un solide qui est cristallisé dans 250 ml de toluène. On obtient 14 g de 2-benzylamino-7-hydroxytétraline base; p.f. 161-163°C.

PREPARATION V

Chlorhydrate de S-2-benzylamino-7-méthoxytétraline.

A une solution de 44 g de 2-benzylamino-7-méthoxytétraline base, PREPARATION I (a), dans 140 ml d'éthanol absolu on ajoute une solution de 24,5 g d'acide (-)mandélique dans 150 ml d'éthanol absolu. Après repos d'une nuit à la température ambiante, on filtre et on lave. On cristallise deux fois dans 250 ml d'éthanol absolu et l'on obtient 33 g de sel pur de l'acide (-)mandélique avec la (-)-2-benzylamino-7-méthoxytétraline; p.f. 155-157°C; $[alpha]_{365}^{20}$ = -316° (méthanol, c = 1%).
Les eaux mères de cette première cristallisation sont séparées et utilisées pour la Préparation VII ci-dessous. On dissout 30 g du sel ainsi obtenu dans 400 ml d'eau et on rend basique la solution avec de l'hydroxyde d'ammonium à 32%. Après extraction de la base par de l'acétate d'éthyle, on lave à l'eau et l'on sèche sur du sulfate de sodium. On évapore le solvant et on obtient 20 g d'une huile que l'on dissout dans de l'isopropanol. On ajoute une solution d'isopropanol saturé d'acide chlorhydrique gazeux et l'on obtient un produit que l'on sèche après filtration. On obtient 22 g d'un produit que l'on cristallise deux fois dans un mélange méthanol/eau 1/1 en obtenant le chlorhydrate de S-2-benzylamino-7-méthoxytétraline; p.f. 287-290°C; $[alpha]_{365}^{20}$ = -231° (méthanol, c = 1%)
La configuration absolue de ce composé (S) a été attribuée en éliminant le groupe N-benzyle et en comparant le pouvoir rotatoire du produit ainsi obtenu avec celui de la littérature.

PREPARATION VI

Bromhydrate de S-2-benzylamino-7-hydroxytétraline

On chauffe au reflux sous agitation pendant 3 heures une solution de 15 g de chlorhydrate de S-2-benzylamino-7-méthoxytétraline dans 100 ml d'acide bromhydrique à 48% et 100 ml d'acide bromhydrique à 33% dans l'acide acétique. On évapore à sec sous pression réduite la solution ainsi obtenue. On reprend le produit trois fois avec de l'éthanol absolu en évaporant chaque fois la solution à sec. On dissout le résidu huileux dans de l'acétone chaude et on cristallise. Après filtration, on lave à l'acétone et ensuite à l'éther éthylique et l'on sèche. On obtient 17 g de bromhydrate de S-2-benzylamino-7-hydroxytétraline; p.f. 198-202°C; $[alpha]_{365}^{20}$ = -201,7° (méthanol, c = 1%).

PREPARATION VII

Chlorhydrate de R-2-benzylamino-7-méthoxytétraline.

On évapore à sec les eaux mères de la première cristallisation du sel de l'acide (-) mandélique avec la (-)-2-benzylamino-7-méthoxytétraline (Préparation V), on met en suspension le résidu aini obtenu dans 400 ml d'eau et on rend la solution basique par de l'hydroxyde d'ammonium à 32%. On extrait la base avec de l'acétate d'éthyle, on lave la solution organique à l'eau, on la sèche sur du sulfate de sodium et on l'évapore à sec. On dissout le résidu ainsi obtenu dans de l'éthanol et on y ajoute une solution de 12.5 g d'acide (+) mandélique dans 75 ml d'éthanol absolu. Après une nuit à la température ambiante, on filtre le précipité obtenu, on le lave et on le cristallise trois fois dans l'éthanol absolu et on obtient 24 g du sel d'addition de l'acide (+) mandélique avec la (+)-2-benzylamino-7-méthoxytétraline; p.f. 152-154°C, $[alpha]_{365}^{20}$ = +309° (méthanol, c = 1%).

On dissout 20 g du sel ainsi obtenu dans 300 ml d'eau et on rend basique la solution aqueuse avec de l'hydroxyde d'ammonium à 32%. On extrait la base avec de l'acétate d'éthyle, on lave la phase organique à l'eau, on la sèche sur du sulfate de sodium et on l'évapore à sec. On dissout le résidu obtenu dans de l'isopropanol et on y ajoute de l'isopropanol saturé d'acide chlorhydrique gazeux, en récupérant le chlorhydrate de R-2-benzylamino-7-méthoxytétraline par filtration.

Le produit ainsi obtenu est séché et cristallisé deux fois dans un mélange méthanol/eau 1/1; p.f. 278-282°C; $[alpha]_{365}^{20}$ = +229,9° (méthanol, c = 1%)

PREPARATION VIII

Bromhydrate de R-2-benzylamino-7-hydroxytétraline.

On dissout 15 g de chlorhydrate de R-2-benzylamino-7-méthoxytétraline dans un mélange de 100 ml d'une solution à 33% d'acide bromhydrique dans l'acide acétique et 100 ml d'acide bromhydrique à 48%, et on chauffe au reflux sous agitation pendant 3 heures la solution ainsi obtenue. Après concentration sous pression réduite, on reprend 3 fois le résidu avec de l'éthanol absolu en séchant chaque fois. On dissout le résidu dans de l'acétone chaude et on le cristallise. On récupère le produit par filtration, on le lave avec de l'acétone et de l'éther éthylique et on le sèche en obtenant 15,5 g de bromhydrate de R-2-benzylamino-7-hydroxytétraline; p.f. 198-202°C, $[alpha]_{365}^{20}$ = +198,4° (méthanol, c = 1%).

EXEMPLE 1

Chlorhydrate de 2-benzylamino-7-(éthoxycarbonylpentan-5-yloxy)tétraline

On chauffe à 70°C sous courant d'azote pendant 30 minutes, 8 g de 2-benzylamino-7-hydroxytétraline base, PREPARATION IV, et 1,7 g d'hydrure de sodium à 55% dans 250 ml de toluène. On laisse refroidir le mélange réactionnel à la température ambiante et on ajoute goutte à goutte un mélange de 10,5 g de 6-bromo-hexanoate d'éthyle et de 0,5 g de bromure de tétrabutylammonium dans 200 ml de toluène, puis on chauffe le mélange réactionnel 8 heures au reflux, on refroidit, on ajoute 100 ml d'eau et on sépare la phase organique. On lave avec une solution d'hydroxyde de sodium 3N, on sèche et on concentre. On solubilise le produit ainsi obtenu dans l'isopropanol et, après avoir ajouté une solution d'isopropanol saturé d'acide chlorhydrique gazeux, on obtient 8,9 g de chlorhydrate de 2-benzylamino-7-(éthoxycarbonylpentan-5-yloxy)tétraline; p.f. 140-142°C; (8.9 g).

EXEMPLE 2

Chlorhydrate de 2-amino-7-(éthoxycarbonylpentan-5-yloxy)tétraline

On soumet une solution de 8,9 g de chlorhydrate de 2-benzylamino-7-(éthoxycarbonylpentan-5-yloxy)tétraline, EXEMPLE 1, dans 150 ml d'éthanol 95% à hydrogénation à la pression atmosphérique et à la température de 60°C en utilisant comme catalyseur 1 g de palladium sur charbon à 10%. Après 3 heures, on filtre, on concentre le filtrat à sec et on reprend le résidu 2 fois avec 100 ml d'éthanol absolu en séchant chaque fois. On triture le produit obtenu dans 100 ml d'acétone, on filtre et on cristallise dans 50 ml d'isopropanol. On obtient 5,5 g de chlorhydrate de 2-amino-7-(éthoxycarbonylpentan-5-yloxy)tétraline; p.f. 114-117°C.

EXEMPLE 3

Chlorhydrate de 2-benzylamino-7-(éthoxycarbonylpropan-3-yloxy)tétraline

On chauffe à 70°C sous courant d'azote pendant 30 minutes, 15 g de 2-benzylamino-7-hydroxytétraline base,

PREPARATION IV, et 2,8 g d'hydrure de sodium à 95% dans 400 ml de toluène. On ajoute goutte à goutte à la température ambiante 9,2 ml de 4-bromobutyrate d'éthyle dans 200 ml de toluène et 0,5 g de bromure de tétrabutylammonium, puis on chauffe le mélange réactionnel 8 heures à 90°C, on refroidit, on extrait deux fois avec 100 ml d'éther éthylique. On lave la phase organique avec un mélange d'hydroxyde de sodium 0,1 N et d'eau et on sèche. On solubilise le produit ainsi obtenu dans 100 ml d'isopropanol et, après avoir ajouté du charbon, on filtre. On rend acide la solution avec de l'isopropanol saturé d'acide chlorhydrique gazeux et on obtient 14 g de chlorhydrate de 2-benzylamino-7-(éthoxycarbonylpropan-3-yloxy)tétraline; p.f. 175-177°C; (14 g).

EXEMPLE 4

Chlorhydrate de 2-amino-7-(éthoxycarbonylpropan-3-yloxy)tétraline

On soumet une solutuion de 14 g de chlorhydrate de 2-benzylamino-7-(éthoxycarbonylpropan-3-yloxy)tétraline, EXEMPLE 3, dans 250 ml d'éthanol 95% et 10 ml d'eau, à hydrogénation à la pression ambiante et à la température de 60°C en utilisant comme catalyseur 2 g de palladium sur charbon à 10%. Après 5 heures, on filtre, on évapore le filtrat à sec et on reprend le résidu plusieurs fois avec de l'éthanol absolu en séchant chaque fois. On triture le produit obtenu dans de l'acétone, on filtre et on cristallise dans de l'isopropanol. On obtient 8,8 g de chlorhydrate de 2-amino-7-(éthoxycarbonylpropan-3-yloxy)tétraline; p.f. 134-136°C.

EXEMPLE 5

Chlorhydrate de 2-benzylamino-7-(éthoxycarbonylbutan-4-yloxy)tétraline

On laisse sous agitation pendant 30 minutes à la température ambiante sous courant d'azote, une solution de 10 g de 2-benzylamino-7-hydroxytétraline base, PREPARATION IV, et 1,6 g d'hydrure de sodium à 80% dans 140 ml de diméthylsulfoxyde. On ajoute au mélange réactionnel ainsi obtenu 10,45 g de 5-bromovalérate d'éthyle et une quantité catalytique d'iodure de potassium. On laisse sous agitation à la température ambiante pendant 17 heures, on ajoute 400 ml d'un mélange eau/glace, et on extrait par de l'acétate d'éthyle. On lave la phase organique avec une solution d'hydroxyde de sodium 2 N et ensuite à l'eau. On sèche sur du sulfate de sodium, on filtre, on concentre sous vide, on dissout le résidu dans de l'isopropanol et on y ajoute de l'isopropanol saturé d'acide chlorhydrique gazeux. Après cristallisation dans 100 ml d'isopropanol, on obtient 10,7 g de chlorhydrate de 2-benzylamino-7-(éthoxycarbonylbutan-4-yloxy)tétraline; p.f. 154-156°C.

EXEMPLE 6

Chlorhydrate de 2-amino-7-(éthoxycarbonylbutan-4-yloxy)tétraline

On soumet une solution de 10,7 g de chlorhydrate de 2-benzylamino-7-(éthoxycarbonylbutan-4-yloxy)tétraline, EXEMPLE 5, dans 250 ml d'éthanol 95% et 25 ml d'eau, à hydrogénation à la pression ambiante et à la température de 60°C en utilisant comme catalyseur 1,2 g de palladium sur charbon à 10%. Après 6 heures, on filtre, on concentre le filtrat à sec et on reprend le résidu plusieurs fois avec de l'éthanol absolu en séchant chaque fois. On triture le produit obtenu dans de l'éther éthylique et on filtre. Après cristallisation dans l'isopropanol, on obtient 8 g de chlorhydrate de 2-amino-7-(éthoxycarbonylbutan-4-yloxy)tétraline; p.f. 131-133°C.

EXEMPLE 7

2-benzylamino-7-(2-carboxypropan-2-yloxy)tétraline et oxalate de 2-benzylamino-7-(2-éthoxycarbonylpropan-2-yloxy)tétraline

On laisse sous agitation pendant 15 minutes à la température ambiante une solution de 19 g de 2-benzylamino-7-hydroxytétraline base, PREPARATION IV, et de 26,6 g de 1,1,1-trichloro-2-méthyl-2-propanol dans 500 ml d'acétone. Après avoir porté la température du mélange réactionnel à 15°C, on ajoute 10,9 g d'hydroxyde de potassium et on laisse sous agitation à la température ambiante pendant 2 heures. Après deux autres additions d'hydroxyde de potassium (10,9 g + 10,9 g), on laisse le mélange réactionnel sous agitation à la température ambiante pendant une nuit. On concentre sous pression réduite, on ajoute 250 ml d'un mélange eau/glace, on lave à l'éther éthylique, on ajoute du charbon, on filtre et on rend acide par de l'acide chlorhydrique jusqu'à pH 5-6. On récupère par filtration 14 g de 2-benzylamino-7-(2-carboxypropan-2-yloxy)tétraline. On chauffe au

reflux pendant 4 heures le produit ainsi obtenu et 3,6 ml de chlorure de thionyle dans 100 ml d'éthanol absolu. Après concentration sous pression réduite, on ajoute un mélange eau/glace et on rend la solution basique par de l'ammoniaque. On extrait par de l'acétate d'éthyle, on sèche, on filtre et on évapore à sec. On solubilise la base ainsi obtenue dans de l'acétone et on ajoute de l'acide oxalique. Après cristallisation du précipité dans 180 ml d'éthanol 95%, on obtient 11,3 g d'oxalate de 2-benzylamino-7-(2-éthoxycarbonylpropan-2-yloxy)tétraline; p.f. 174-176°C.

Un échantillon de 2-benzylamino-7-(2-carboxypropan-2-yloxy)tétraline obtenue comme décrit ci-dessus, est lavé à l'eau, trituré dans de l'acétone et séché; p.f. 240-242°C.

Par hydrogénation dudit composé selon la procédure de l'Exemple 2, on obtient le produit correspondant déprotégé, la 2-amino-7-(2-carboxypropan-2-yloxy)tétraline.

EXEMPLE 8

Oxalate de 2-amino-7-(2-éthoxycarbonylpropan-2-yloxy)tétraline

On soumet une solution de 9,1 g de 2-benzylamino-7-(2-éthoxycarbonylpropan-2-yloxy)tétraline base, EXEMPLE 7, dans 100 ml d'éthanol 95% et 4 ml d'acide chlorhydrique, à hydrogénation à la pression ambiante et à la température de 60°C en utilisant comme catalyseur 1 g de palladium sur charbon à 10%. Après 4 heures, on filtre, on concentre sous pression réduite et on reprend le résidu plusieurs fois par de l'éthanol absolu en séchant chaque fois. Le résidu est solubilisé dans de l'ammoniaque diluée. Après extraction par de l'éther éthylique, on sépare, on rend anhydre, on filtre et on évapore à sec. On purifie par flash-chromatographie en utilisant un mélange chlorure de méthylène/éthanol 8/2 et on solubilise le produit ainsi obtenu dans de l'acétone contenant de l'acide oxalique. Après cristallisation dans 10 ml d'acétone, on obtient 0,66 g d'oxalate de 2-amino-7-(2-éthoxycarbonylpropan-2-yloxy)tétraline; p.f. 140-142°C.

EXEMPLE 9

Chlorhydrate de S-2-benzylamino-7-(2-éthoxycarbonylpropan-2-yloxy)tétraline

Le composé indiqué dans le titre a été obtenu en suivant sensiblement le même procédé que dans l'Exemple 7, mais en partant de la S-2-benzylamino-7-hydroxytétraline base (préparée à partir du bromhydrate correspondant décrit dans la Préparation VII, par dissolution de ce produit dans l'eau, addition d'une solution concentrée de hydroxyde d'ammonium pour rendre la solution basique, extraction de la base libre avec de l'acétate d'éthyle et évaporation du solvant). A la fin de ce procédé, la base libre obtenue est dissoute dans de l'isopropanol et de l'isopropanol saturé en acide chlorhydrique gazeux est ajouté pour faire précipiter le chlorhydrate qui est séparé par filtration et cristallisé dans l'isopropanol.

[alpha] $^{20}_{365}$ = - 156,2° (méthanol, c = 0,5%) p.f. 152-154°C

EXEMPLE 10

Chlorydrate de R-2-benzylamino-7-(2-éthoxycarbonylpropan-2-yloxy)tétraline

Le composé indiqué dans le titre a été obtenu en suivant presque le même procédé de l'Exemple 7, mais en partant de la R-2-benzylamino-7-hydroxytétraline base (préparée à partir du bromhydrate correspondant décrit dans la Préparation VIII, par dissolution de ce produit dans l'eau, addition d'une solution concentrée de hydroxyde d'ammonium pour rendre la solution basique, extraction de la base libre avec de l'acétate d'éthyle et évaporation du solvant). A la fin de ce procédé, la base libre obtenue est dissoute dans de l'isopropanol et de l'isopropanol saturé en acide chlorhydrique gazeux est ajouté pour faire précipiter le chlorhydrate qui est séparé par filtration et cristallisé dans l'isopropanol.

[alpha]$^{20}_{365}$ = + 158,4° (méthanol, c = 0,5 %) p.f. 148-150°C

EXEMPLE 11

Oxalate de S-2-amino-7-(2-éthoxycarbonylpropan-2-yloxy)tétraline

Le composé indiqué dans le titre a été obtenu en suivant le procédé de l'Exemple 8, mais en partant du composé obtenu dans l'Exemple 9.

$[alpha]^{20}_{365}$ = - 140,4° (méthanol, c = 1%) p.f. : 132-134°C

EXEMPLE 12

Oxalate de R-2-amino-7-(2-éthoxycarbonylpropan-2-yloxy)tétraline

Le composé indiqué dans le titre a été obtenu en suivant la procédure de l'Exemple 8, mais en partant du composé obtenu dans l'Exemple 10.
$[alpha]^{20}_{365}$ =+140,9° (méthanol, c = 1%) p.f. : 131-134°C

EXEMPLE 13

Chlorhydrate de N-[7-(éthoxycarbonylbutan-4-yloxy)-1,2,3,4-tétrahydronapht-2-yl]-2-hydroxy-2-(3-chlorophényl)éthanamine

On chauffe à 80°C sous agitation sous courant d'azote pendant 11 heures une solution de 5 g de 2-amino-7-(éthoxycarbonylbutan-4-yloxy)tétraline base (obtenue par dissolution de son chlorhydrate décrit dans l'Exemple 6, dans l'eau, addition d'une solution concentrée d'hydroxyde d'ammonium jusqu'à pH basique, extraction de la base libre avec de l'acétate d'éthyle et évaporation du solvant organique), dans 15 ml de diméthylsulfoxyde anhydre et 4,6 g d'oxyde de 3-chlorostyrène. Après repos d'une nuit à la température ambiante, on verse le mélange réactionnel dans un mélange eau/glace, on extrait par de l'acétate d'éthyle, on lave à l'eau et on sèche sur du sulfate de sodium. Après filtration, on évapore à sec, on dissout le résidu ainsi obtenu dans de l'éther éthylique et on rend acide la solution par de l'isopropanol saturé d'acide chlorhydrique. On filtre et on sèche. On cristallise deux fois dans 40 ml d'isopropanol et on obtient 3,9 g de chlorhydrate de N-[7-(éthoxycarbonylbutan-4-yloxy)-1,2,3,4-tétrahydronapht-2-yl]-2-hydroxy-2-(3-chlorophényl) éthanamine; p.f. 127-130°C.

EXEMPLE 14

Chlorhydrate de N-[7-(éthoxycarbonylpentan-5-yloxy)-1,2,3,4-tétrahydronapht-2-yl]-2-hydroxy-2-(3-chlorophényl)éthanamine

On agite à 25°C pendant 20 minutes une solution de 4,2 g de 2-amino-7-(éthoxycarbonylpentan-5-yloxy)tétraline base, obtenue par neutralisation de son chlorhydrate décrit dans l'Exemple 2, et 2,4 g de triméthylsilylacétamide dans 10 ml de diméthylsulfoxyde anhydre et on y ajoute 3,6 g d'oxyde de 3-chlorostyrène. On chauffe le mélange réactionnel à 80°C pendant 9 heures et après on le verse dans 100 ml d'eau contenant 3 ml d'acide chlorhydrique concentré. On y ajoute 50 ml d'acétate d'éthyle et on agite le mélange pendant 1 heure. On sépare les deux phases: on lave la phase aqueuse avec de l'acétate d'éthyle (2 x 50 ml) et on ajoute les liquides de lavage à la phase organique. On lave cette solution organique avec de l'eau, une solution diluée d'hydroxyde d'ammonium et à nouveau avec de l'eau, on la sèche et on évapore à sec. On purifie le résidu par flash chromatographie en utilisant de l'acétate d'éthyle. On dissout le résidu obtenu par évaporation à sec des fractions réunies dans 100 ml d'éther isopropylique et on y ajoute de l'isopropanol saturé d'acide chlorhydrique gazeux. Le produit huileux qui se sépare, solidifie par repos et il est récupéré par filtration (3,9 g). On cristallise ce produit dans 20 ml d'isopropanol en obtenant 2 g du composé indiqué dans le titre; p.f. 109-112°C.

EXEMPLE 15

Chlorhydrate de N-[7-(éthoxycarbonylpropan-3-yloxy)-1,2,3,4-tétrahydronapht-2-yl]-2-hydroxy-2-(3-chlorophényl)éthanamine

On maintient à 25°C pendant 20 minutes sous courant d'azote un mélange de 5,3 g de 2-amino-7-(éthoxycarbonylpropan-3-yloxy)tétraline base, obtenue par néutralisation de son chlorhydrate décrit dans l'Exemple 4, et 2,75 g de N-triméthylsilylacétamide dans 10 ml de diméthylsulfoxyde anhydre. On y ajoute 3 g d' oxyde de 3-chlorostyrène et on chauffe le mélange réactionnel à 60°C pendant 7 heures. Après repos d'une nuit à la température ambiante, on verse la mélange réactionnel dans de l'eau et on extrait par de l'acétate d'éthyle. On lave à fond la phase organique à l'eau, on sèche et on évapore à sec. On dissout le résidu dans de l'isopropanol et on y ajoute de l'isopropanol saturé d'acide chlorhydrique gazeux. On récupère le précipité par filtration et on le cristallise dans 80 ml d'isopropanol on obtient 4,4 g du composé indiqué dans le titre; p.f. 156-158°C.

EXEMPLE 16

Chlorhydrate de N-[7-(2-éthoxycarbonylpropan-2-yloxy)-1,2,3,4-tétrahydronapht-2-yl]-2-hydroxy-2-(3-chlorophényl)éthanamine

On agite à la température ambiante sous courant d'azote et dans des conditions anhydres pendant 20 minutes un mélange de 3,2 g de 2-amino-7-(2-éthoxycarbonylpropan-2-yloxy)tétraline base, obtenue par neutralisation de son oxalate décrit dans l'Exemple 8, et 2,5 g de N-triméthylsilylacétamide dans 10 ml de diméthylsulfoxyde anhydre et on y ajoute 2,8 g d'oxyde de 3-chlorostyrène. On chauffe le mélange réactionnel à 80°C pendant 8 heures et, après repos d'une nuit à la température ambiante, on y ajoute encore 1,0 g d'oxyde de 3-chlorostyrène, on chauffe à 80°C pendant 5 heures et on verse le mélange réactionnel dans 150 ml d'un mélange eau/glace. On ajoute quelques millilitres d'acide chlorhydrique concentré et 100 ml d'acétate d'éthyle et on agite le mélange ainsi obtenu à la température ambiante pendant 1 heure. On sépare les deux phases, on lave la phase aqueuse deux fois avec 50 ml d'acétate d'éthyle chaque fois et on ajoute ces deux portions d'acétate d'éthyle de lavage à la phase organique séparée. On lave la phase organique à l'eau, à une solution diluée d'hydroxyde d'ammonium et ensuite à l'eau, on sèche et on évapore à sec en obtenant 6,6 g de produit huileux. On purifie ce produit par flash chromatographie en utilisant de l'acétate d'éthyle et on transforme le produit purifié ainsi obtenu dans son chlorhydrate en le dissolvant dans de l'éther isopropylique et en rendant acide la solution par addition d'isopropanol saturé d'acide chlorhydrique gazeux. Après repos, on récupère par filtration le précipité qui s'est formé, et on le cristallise deux fois dans de l'isopropanol; on obtient 1 g du composé indiqué dans le titre; p.f. 142-144°C.

EXEMPLE 17

Chlorhydrate de N-[(2S)-7-(2-éthoxycarbonylpropan-2-yloxy)-1,2,3,4-tétrahydronapht-2-yl]-(2R)-2-hydroxy-2-(3-chlorophényl)éthanamine

Le composé indiqué dans le titre a été préparé selon le procédé de l'Exemple 16, mais en partant de l'oxyde de (R)-3-chlorostyrène et de la S-2-amino-7-(2-éthoxycarbonylpropan-2-yloxy)tétraline base obtenue par neutralisation de l'oxalate correspondant décrit dans l'Exemple 11.
$[alpha]_{365}^{20}$ = - 270° (méthanol, c = 1%) ; p.f. : 206-208°C

EXEMPLE 18

Chlorhydrate de N-[(2R)-7-(2-éthoxycarbonylpropan-2-yloxy)-1,2,3,4-tétrahydronapht-2-yl]-(2R)-2-hydroxy-2-(3-chlorophényl)éthanamine

Le composé indiqué dans le titre a été préparé selon le procédé de l'Exemple 16, mais en partant de l'oxyde de (R)-3-chlorostyrène et de la R-2-amino-7-(2-éthoxycarbonylpropan-2-yloxy)tétraline base obtenue par neutralisation de l'oxalate correspondant décrit dans l'Exemple 12.
$[alpha]_{365}^{20}$ =+ 125,7°(méthanol, c = 1%) ; p.f. : 109-112°C

Les composés des Exemples 13 et 16 ont été évalués dans le test du côlon isolé de rat qui a été conduit selon la méthode décrite dans la demande de brevet EP-A-255415.

Ces produits ont montré une activité, en termes de CI50, supérieure à celle de l'oxalate de N-[7-(éthoxycarbonylméthoxy)-1,2,3,4-tétrahydronapht-2-yl]-2-hydroxy-2-(3-chlorophenyl)éthanamine décrit dans l'Exemple 11 de EP 211721.

## Revendications

### Revendications pour les Etats contractants suivants : BE, CH, DE, DK, FR, GB, LI, LU, NL, SE, AT

**1.** Un carboxyalkyl-éther de la 2-amino-7-hydroxytétraline de formule

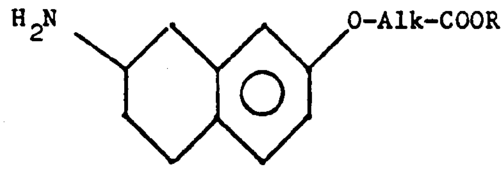

I

dans laquelle Alk représente un groupe alkylène de 3 à 5 atomes de carbone à chaîne droite ou ramifiée, et R représente l'hydrogène ou un groupe alkyle de 1 à 4 atomes de carbone, ou un de ses sels.

**2.** Composé selon la revendication 1,dans laquelle Alk représente
(a) un groupe

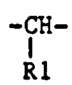 , 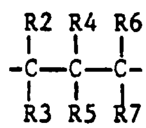 , 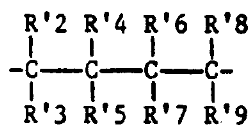 ,

ou $-(CH_2)_5-$
où
R1 est une groupe éthyle, propyle ou butyle ;
R2 à R7 sont tous des hydrogènes ou un des radicaux R2 à R7 est un groupe méthyle ou éthyle et les autres sont des hydrogènes ou deux des radicaux R2 à R7 sont des groupes méthyle et les autres sont des hydrogènes ;
R'2 à R'9 sont tous des hydrogènes ou un des radicaux R'2 à R'9 est un groupe méthyle et les autres sont des hydrogènes;
(b) un groupe

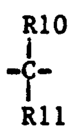

où
R10 et R11 représentent indépendamment un groupe méthyle ou éthyle ; ou
R11 est aussi le groupe propyle lorsque R10 est méthyle ;
(c) un groupe

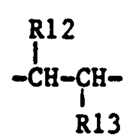

où l'un de R12 et R13 est un atome d' hydrogène et l'autre est un groupe méthyle, éthyle ou propyle, ou l'un de R12 et R13 est le groupe méthyle et l'autre est un groupe méthyle ou éthyle.

**3.** Composé selon la revendication 2,dans laquelle Alk représente un groupe

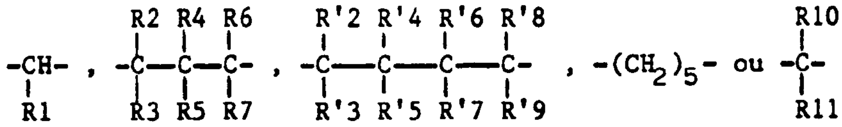

où $R_1$-$R_7$, $R_{10}$, $R_{11}$ et $R'_2$-$R'_9$ sont tels que définis dans la revendication 2.

4. Composé selon la revendication 3 qui est choisi parmi la 2-amino-7-(éthoxycarbonylpropan-3-yloxy)-tétraline, la 2-amino-7-(éthoxycarbonylbutan-4-yloxy)-tétraline, la 2-amino-7-(éthoxycarbonylpentan-5-yloxy)-tétraline, la 2-amino-7-(2-éthoxycarbonylpropan-2-yloxy)-tétraline et leurs sels.

5. Un procédé pour la préparation d'un carboxyalkyl-éther de la 2-amino-7-hydroxytétraline de formule (I)

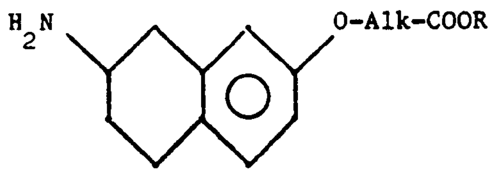

(I)

dans laquelle Alk et R sont tels que définis dans la revendication 1 ou d'un de ses sels, caractérisé en ce que

(A) on soumet une 2-amino-7-hydroxytétraline N-protégée de formule (II)

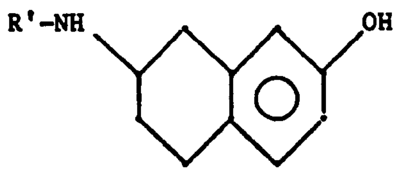

(II)

dans laquelle R' est un groupe protecteur du groupe amino qui peut être éliminé par hydrogénation catalytique ou par hydrolyse acide douce, à une réaction de carb(alk)oxyalkylation avec un composé de formule (IIIa)

$$Hal - Alk^1 - COOR \quad (IIIa)$$

dans laquelle R est tel que défini ci-dessus, Hal représente un atome de chlore, brome ou iode, et $Alk^1$ représente un groupe

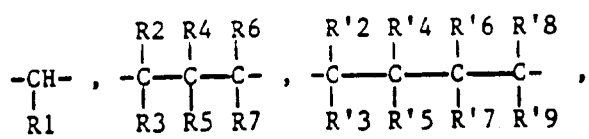

ou $-(CH_2)_5-$

où

R1 est un groupe éthyle, propyle ou butyle ;

R2 à R7 sont tous des hydrogènes ou un des radicaux R2 à R7 est un groupe méthyle ou éthyle et les autres sont des hydrogènes ou deux des radicaux R2 à R7 sont des groupes méthyle et les autres sont des hydrogènes ;

R'2 à R'9 sont tous des hydrogènes ou un des radicaux R'2 à R'9 est un groupe méthyle et les autres sont des hydrogènes ;

en présence d'un agent de condensation basique; ou avec un composé de formule (IIIb)

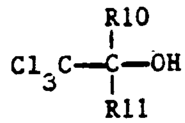

(IIIb)

dans laquelle
R10 et R11 représentent indépendamment un groupe méthyle ou éthyle, ou R11 est aussi le groupe propyle lorsque R10 est le groupe méthyle ;
en présence d'une base forte, suivie éventuellement d'une réaction du composé ainsi obtenu avec du chlorure de thionyle dans un alcanol en $C_1$-$C_4$ convenablement choisi;
ou avec un composé de formule (IIIc)

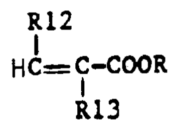

(IIIc)

dans laquelle R est tel que défini ci-dessus et l'un de R12 et R13 est l'hydrogène et l'autre est un groupe méthyle, éthyle ou propyle ou l'un de R12 et R13 est le groupe méthyle et l'autre est le groupe méthyle ou éthyle, éventuellement en présence de quantités catalytiques d'un hydroxyde d'ammonium quaternaire;
pour obtenir une 2-amino-7-hydroxytétraline N-protégée de formule (IV)

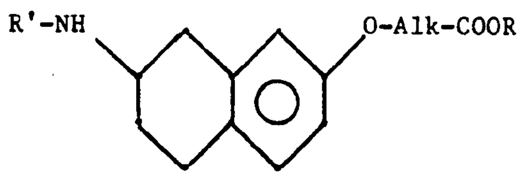

(IV)

dans laquelle Alk, R et R' sont tels que définis ci-dessus, et
(B) on élimine le groupe N-protecteur par hydrogénation catalytique ou par hydrolyse acide douce et, après avoir éventuellement saponifié le groupe carbalcoxy inférieur en carboxy, on isole le composé de formule (I), sous forme de base libre ou de sel, et on le transforme éventuellement en l'un de ses sels.

6. Procédé selon la revendication 5 caractérisé en ce que le groupe N-protecteur R' est choisi parmi les groupes tert-butoxycarbonyle, benzyloxycarbonyle, et les groupes benzyle, benzhydryle et trityle, non substitués ou substitués par un groupe méthoxy sur un noyau phényle.

7. Un carboxyalkyl-éther d'une 2-amino-7-hydroxytétraline N-protégée de formule (IV)

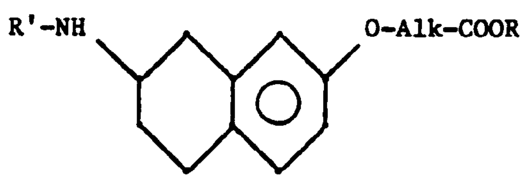

(IV)

dans laquelle R, Alk et R' sont tels que définis dans la revendication 5, ou un de ses sels.

8. Composé selon la revendication 7 où R' est tel que défini dans la revendication 6.

9. Composé selon la revendication 8, choisi parmi la 2-benzylamino-7-(éthoxycarbonylpropan-3-yloxy)tétraline, la 2-benzylamino-7-(éthoxycarbonylbutan-4-yloxy)tétraline, la 2-benzylamino-7-(éthoxycarbonylpentan-5-yloxy)tétraline, la 2-benzylamino-7-(2-éthoxycarbonylpropan-2-yloxy)tétraline, et leurs sels.

**10.** Une phényléthanolaminotétraline de formule (XII)

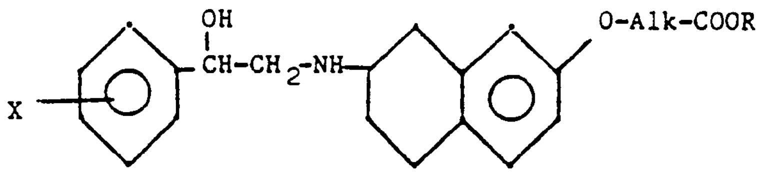

(XII)

dans laquelle
X représente l'hydrogène, un halogène, un groupe alkyle en $C_1$-$C_4$ ou le groupe trifluorométhyle,
R représente l'hydrogène ou un groupe alkyle en $C_1$-$C_4$ et Alk est tel que défini dans l'une quelconque des revendication 1 à 3, et ses sels pharmaceutiquement acceptables.

**11.** Un procédé pour la préparation d'une phényléthanolaminotétraline de la revendication 10 caractérisé en ce qu'on fait réagir un composé de formule (I)

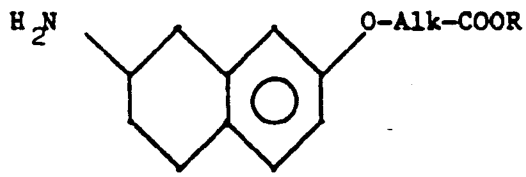

(I)

dans laquelle Alk représente un groupe alkylène de 3 à 5 atomes de carbone à chaîne droite ou ramifiée, et R représente l'hydrogène ou un groupe alkyle de 1 à 4 atomes de carbone, avec un époxyde de styrène de formule (XIII)

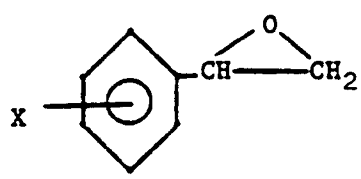

(XIII)

dans laquelle X est tel que défini dans la revendication 10,
ou avec un dérivé fonctionnel d'un acide mandélique de formule (XIV)

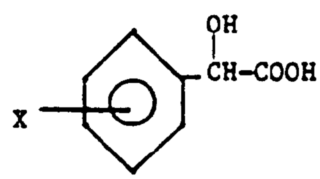

(XIV)

dans laquelle X est tel que défini ci-dessus et on reduit le groupe carbonyle amidique du mandélamide intermédiaire ainsi obtenu de formule

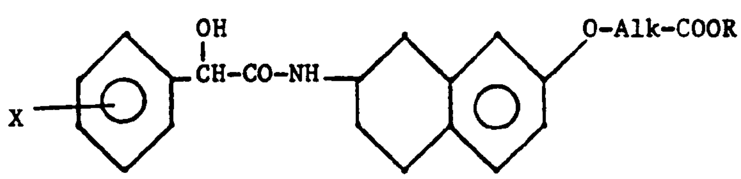

XV

dans laquelle X, Alk et R sont tels que définis ci-dessus, en groupe méthylène
et éventuellement on transforme le produit de formule (XII) ainsi obtenu dans un de ses sels pharmaceutiquement acceptables.

**12.** Composé selon la revendication 10 dans laquelle Alk représente un groupe

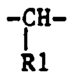 , 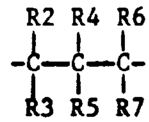 , 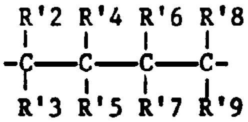 , $-(CH_2)_5-$ ou 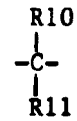

où
R1 est un groupe éthyle, propyle ou butyle ;
R2 à R7 sont tous des hydrogènes ou un des radicaux R2 à R7 est un groupe méthyle ou éthyle et les autres sont des hydrogènes ou deux des radicaux R2 à R7 sont des groupes méthyle et les autres sont des hydrogènes ;
R'2 à R'9 sont tous des hydrogènes ou un des radicaux R'2 à R'9 est un groupe méthyle et les autres sont des hydrogènes;
R10 et R11 représentent indépendamment un groupe méthyle ou éthyle, ou R11 est aussi le groupe propyle lorsque R10 est le groupe méthyle ;

**13.** Composé selon la revendication 12 dans laquelle Alk représente un groupe

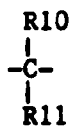

où R10 et R11 sont tels que définis dans la revendication 12.

**14.** Composé selon la revendication 10, qui est la N-[7-(éthoxycarbonylbutan-4-yloxy)-1,2,3,4-tétrahydronapht-2-yl]-2-hydroxy-2-(3-chlorophényl)éthanamine et ses sels pharmaceutiquement acceptables.

**15.** Composé selon la revendication 10 qui est la N-[7-(éthoxycarbonylpropan-3-yloxy)-1,2,3,4-tétrahydronapht-2-yl]-2-hydroxy-2-(3-chlorophényl)éthanamine et ses sels pharmaceutiquement acceptables.

**16.** Composé selon la revendication 10 qui est la N-[7-(éthoxycarbonylpentan-5-yloxy)-1,2,3,4-tétrahydronapht-2-yl]-2-hydroxy-2-(3-chlorophényl)éthanamine et ses sels pharmaceutiquement acceptables.

**17.** Composé selon la revendication 10 qui est la N-[7-(2-éthoxycarbonylpropan-2-yloxy)-1,2,3,4-tétrahydronapht-2-yl]-2-hydroxy-2-(3-chlorophényl)éthanamine et ses sels pharmaceutiquement acceptables.

**18.** Composition pharmaceutique pour le traitement des troubles gastrointestinaux caractérisée en ce qu'elle contient un composé de la revendication 10 en tant que principe actif.

**Revendications pour l'Etat contractant suivant : ES**

**1.** Procédé pour la préparation d'un carboxyalkyl-éther de la 2-amino-7-hydroxytétraline de formule (I)

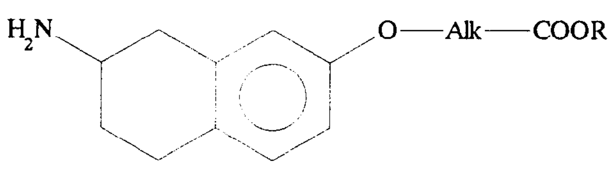

I

dans laquelle Alk représente un groupe alkylène de 3 à 5 atomes de carbone à chaîne droite ou ramifiée, et R représente l'hydrogène ou un groupe alkyle de 1 à 4 atomes de carbone, ou d'un de ses sels, caractérisé en ce que

(A) on soumet une 2-amino-7-hydroxytétraline N-protégée de formule (II)

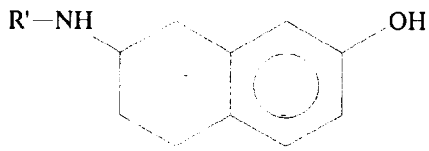

(II)

dans laquelle R' est un groupe protecteur du groupe amino qui peut être éliminé par hydrogénation catalytique ou par hydrolyse acide douce, à une réaction de carb(alk)oxyalkylation avec un composé de formule (IIIa)

$$\text{Hal - Alk}^1\text{ - COOR} \quad \text{(IIIa)}$$

dans laquelle R est tel que défini ci-dessus, Hal représente un atome de chlore, brome ou iode, et $Alk^1$ représente un groupe

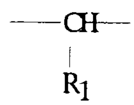 , 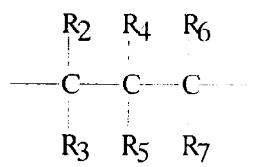 , 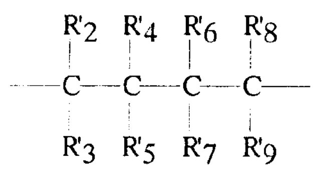

ou-$(CH_2)_5$ -
où
$R_1$ est un groupe éthyle, propyle ou butyle ;
$R_2$ à $R_7$ sont tous des hydrogènes ou un des radicaux $R_2$ à $R_7$ est un groupe méthyle ou éthyle et les autres sont des hydrogènes ou deux des radicaux $R_2$ à $R_7$ sont des groupes méthyle et les autres sont des hydrogènes ;
$R'_2$ à $R'_9$ sont tous des hydrogènes ou un des radicaux $R'_2$ à $R'_9$ est un groupe méthyle et les autres sont des hydrogènes ;
en présence d'un agent de condensation basique ;
ou avec un composé de formule (IIIb)

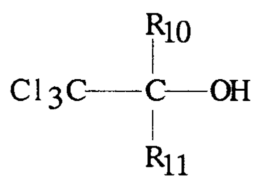

(III b )

dans laquelle
$R_{10}$ et $R_{11}$ représentent indépendamment un groupe méthyle ou éthyle, ou $R_{11}$ est aussi le groupe propyle lorsque $R_{10}$ est le groupe méthyle; en présence d'une base forte, suivie éventuellement d'une réaction du composé ainsi obtenu avec du chlorure de thionyle dans un alcanol en $C_1$-$C_4$ convenablement choisi;
ou avec un composé de formule (IIIc)

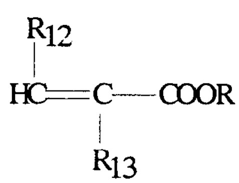

(IIIc)

dans laquelle R est tel que défini ci-dessus et l'un de $R_{12}$ et $R_{13}$ est l'hydrogène et l'autre est un groupe méthyle, éthyle ou propyle ou l'un de $R_{12}$ et $R_{13}$ est le groupe méthyle et l'autre est le groupe méthyle ou éthyle, éventuellement en présence de quantités catalytiques d'un hydroxyde d'ammonium quaternaire ;
pour obtenir une 2-amino-7-hydroxytétraline N-protégée de formule (IV)

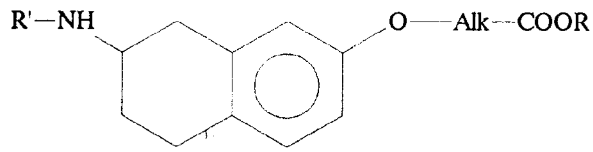

(IV)

dans laquelle Alk, R et R' sont tels que définis ci-dessus, et
(B) on élimine le groupe N-protecteur par hydrogénation catalytique ou par hydrolyse acide douce et, après avoir éventuellement saponifié le groupe carbalcoxy inférieur en carboxy, on isole le composé de formule (I), sous forme de base libre ou de sel, et on le transforme éventuellement en l'un de ses sels.

2. Procédé selon la revendication 1, caractérisé en ce que le groupe N-protecteur R' est choisi parmi les groupes tert-butoxycarbonyle, benzyloxycarbonyle, et les groupes benzyle, benzhydryle et trityle, non substitués ou substitués par un groupe méthoxy sur un noyau phényle.

3. Procédé selon l'une des revendications 1 ou 2, caractérisé en ce que Alk représente un groupe

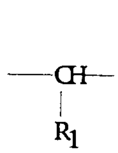 , 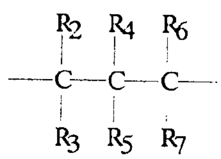 , 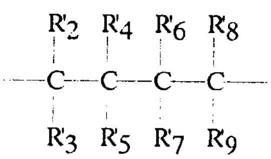 , $-(CH_2)_5-$ ou 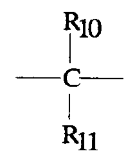

dans lesquels les groupes $R_1$-$R_7$, $R_{10}$, $R_{11}$ et $R'_2$-$R'_9$ sont tels que définis dans la revendication 1.

4. Procédé selon la revendication 3, caractérisé en ce que l'on fait réagir un composé de formule (II) dans laquelle R' est l'hydrogène avec
  * soit un composé de formule (IIIa) dans-laquelle $Alk^1$ est : un groupe

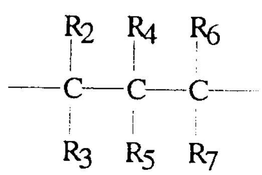 , 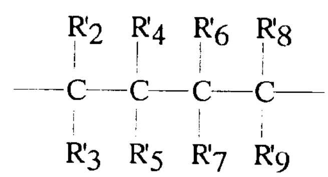

ou $-(CH_2)_5-$
où $R_2$-$R_7$ et $R'_2$-$R'_9$ représentent chacun un hydrogène,
et R représente un groupe éthyle;
  * soit un composé de formule (IIIb) dans laquelle $R_{10}$ et $R_{11}$ sont un groupe méthyle pour former les composés ci-après :
la 2-amino-7-(éthoxycarbonylpropan-3-yloxy)-tétraline,
la 2-amino-7-(éthoxycarbonylbutan-4-yloxy)-tétraline,
la 2-amino-7-(éthoxycarbonylpentan-5-yloxy)-tétraline,
la 2-amino-7-(2-éthoxycarbonylpropan-2-yloxy)-tétraline
et leurs sels.

5. Procédé pour la préparation d'un carboxyalkyl-éther d'une 2-amino-7-hydroxytétraline N-protégée de formule (IV)

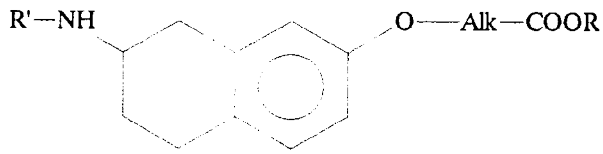

(IV)

dans laquelle R, Alk et R' sont tels que définis dans la revendication 1, ou d'un de ses sels, caractérisé en ce que :

(A) on soumet une 2-amino-7-hydroxytétraline N-protégée de formule (II)

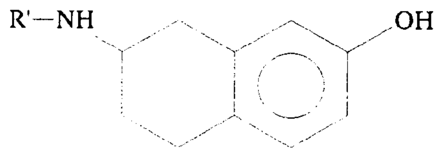

dans laquelle R' est un groupe protecteur du groupe amino qui peut être éliminé par hydrogénation catalytique ou par hydrolyse acide douce, à une réaction de carb(alk)oxyalkylation avec un composé de formule (IIIa) :

Hal - $Alk^1$ - COOR (IIIa)

dans laquelle R est tel que défini ci-dessus, Hal représente un atome de chlore, brome ou iode, et $Alk^1$ représente un groupe

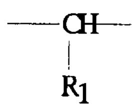 , 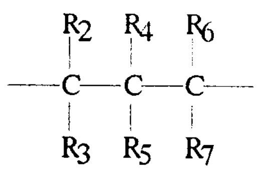 , 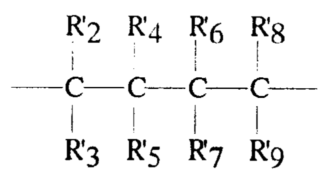

ou $-(CH_2)_5-$

où

$R_1$ est un groupe éthyle, propyle ou butyle;

$R_2$ à $R_7$ sont tous des hydrogènes ou un des radicaux $R_2$ à $R_7$ est un groupe méthyle ou éthyle et les autres sont des hydrogènes ou deux des radicaux $R_2$ à $R_7$ sont des groupes méthyle et les autres sont des hydrogènes;

$R'_2$ à $R'_9$ sont tous des hydrogènes ou un des radicaux $R'_2$ à $R'_9$ est un groupe méthyle et les autres sont des hydrogènes;

en présence d'un agent de condensation basique;

ou avec un composé de formule (IIIb)

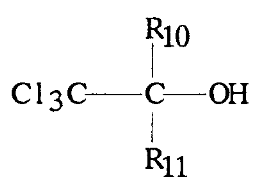

(III b)

dans laquelle

$R_{10}$ et $R_{11}$ représentent indépendamment un groupe méthyle ou éthyle, ou $R_{11}$ est aussi le groupe propyle lorsque $R_{10}$ est le groupe méthyle;

en présence d'une base forte, suivie éventuellement d'une réaction du composé ainsi obtenu avec du chlorure de thionyle dans un alcanol en $C_1$-$C_4$ convenablement choisi;

ou avec un composé de formule (IIIc)

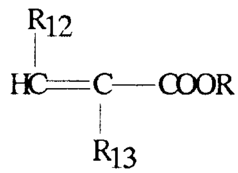

(IIIc)

dans laquelle R est tel que défini ci-dessus et l'un de $R_{12}$ et $R_{13}$ est l'hydrogène et l'autre est un groupe méthyle, éthyle ou propyle, ou l'un de $R_{12}$ et $R_{13}$ est le groupe méthyle et l'autre est le groupe méthyle ou éthyle, éventuellement en présence de quantités catalytiques d'un hydroxyde d'ammonium quaternaire, et on le transforme éventuellement en l'un de ses sels.

**6.** Procédé selon la revendication 5, caractérisé en ce que R' est tel que défini dans la revendication 2.

**7.** Procédé selon la revendication 6, caractérisé en ce que l'on fait réagir un composé de formule (II) dans laquelle R' est un groupe benzyle avec

- soit un composé de formule (IIIa) dans laquelle $Alk^1$ représente un groupe

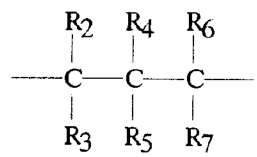

,

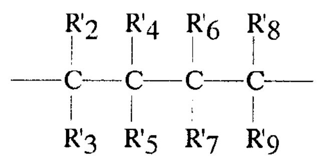

ou $-(CH_2)_5-$
où $R_2$-$R_7$ et $R'_2$-$R'_9$ représentent chacun un hydrogène,
et R représente un groupe éthyle;

- soit un composé de formule (IIIc) dans laquelle $R_{12}$ est un groupe méthyle, $R_{13}$ est un hydrogène et R est un groupe éthyle, pour former les composés ci-après :
la 2-benzylamino-7-(éthoxycarbonylpropan-3-yloxy)tétraline,
la 2-benzylamino-7-(éthoxycarbonylbutan-4-yloxy)tétraline,
la 2-benzylamino-7-(éthoxycarbonylpentan-5-yloxy)tétraline,
la 2-benzylamino-7-(éthoxycarbonylpropan-2-yloxy)tétraline,
et leurs sels.

**8.** Procédé pour la préparation d'une phényléthanolaminotétraline de formule (XII)

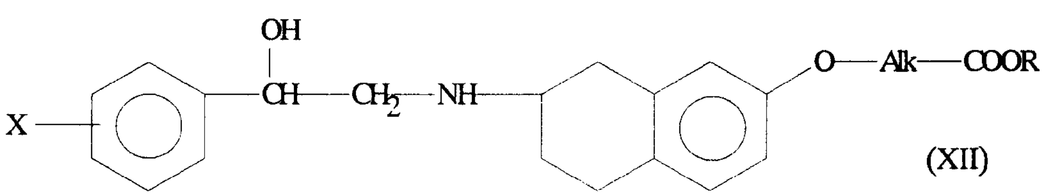

(XII)

dans laquelle
X représente l'hydrogène, un halogène, un groupe alkyle en $C_1$-$C_4$ ou le groupe trifluorométhyle,
R représente l'hydrogène ou un groupe alkyle en $C_1$-$C_4$ et Alk est tel que défini dans l'une des revendications 1 ou 3, et de ses sels pharmaceutiquement acceptables, caractérisé en ce que l'on fait réagir un composé de formule (I)

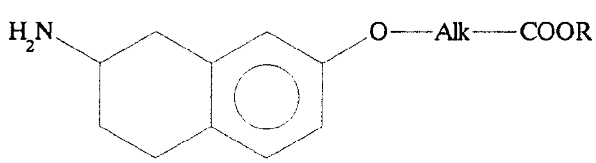

I

dans laquelle Alk représente un groupe alkylène de 3 à 5 atomes de carbone à chaîne droite ou ramifiée, et R est tel que défini ci-dessus,

* soit avec un époxyde de styrène de formule (XIII)

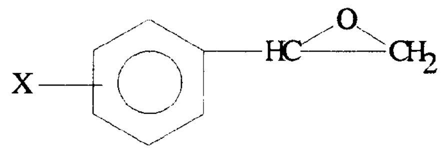

(XIII)

dans laquelle X est tel que défini ci-dessus,

* soit avec un dérivé fonctionnel d'un acide mandélique de formule (XIV)

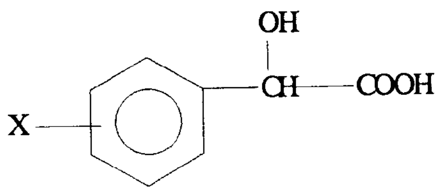

(XIV)

dans laquelle X est tel que défini ci-dessus et on réduit le groupe carbonyle amidique du mandélamide intermédiaire ainsi obtenu de formule

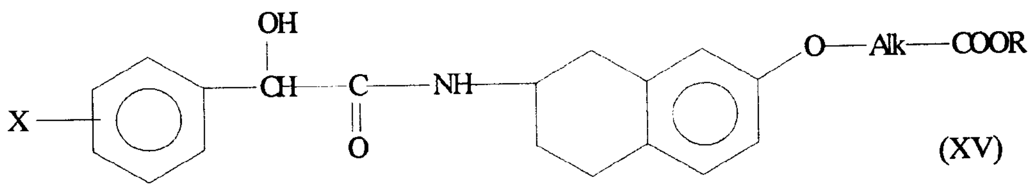

(XV)

dans laquelle X, Alk et R sont tels que définis ci-dessus, en groupe méthylène, et éventuellement on transforme le produit de formule (XII) ainsi obtenu en l'un de ses sels pharmaceutiquement acceptables.

**9.** Procédé selon la revendication 8, caractérisé en ce que Alk représente un groupe

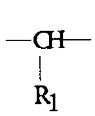 , 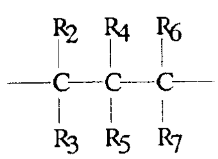 , 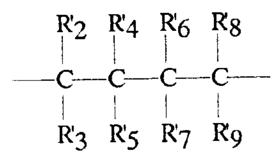 , $-(CH_2)_5-$ ou 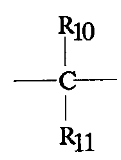

où

$R_1$ est un groupe éthyle, propyle ou butyle ;

$R_2$ à $R_7$ sont tous des hydrogènes ou un des radicaux $R_2$ à $R_7$ est un groupe méthyle ou éthyle et les autres sont des hydrogènes ou deux des radicaux $R_2$ à $R_7$ sont des groupes méthyle et les autres sont des hydrogènes ;

$R'_2$ à $R'_9$ sont tous des hydrogènes ou un des radicaux $R'_2$ à $R'_9$ est un groupe méthyle et les autres sont des hydrogènes ;

$R_{10}$ et $R_{11}$ représentent indépendamment un groupe méthyle ou éthyle, ou $R_{11}$ est aussi le groupe propyle lorsque $R_{10}$ est le groupe méthyle.

**10.** Procédé selon la revendication 9, caractérise en ce que Alk représente un groupe

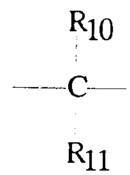

où $R_{10}$ et $R_{11}$ sont tels que définis dans la revendication 9.

**11.** Procédé selon la revendication 8, pour la préparation de la N-[7-(éthoxycarbonylbutan-4-yloxy)- 1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe tétraméthylène et R est un groupe éthyle avec soit un composé de formule (XIII) dans laquelle X est un groupe 3-chloro, soit un composé de formule (XIV) dans laquelle X est un groupe 3-chloro.

**12.** Procédé selon la revendication 8, pour la préparation de la N-[7-(éthoxycarbonylpropan-3-yloxy)- 1, 2, 3, 4-tétrahydronapht-2-yl]-2-hydroxy-2-(3-chlorophényl)éthanamie et de ses sels pharmaceutiquement acceptables, caractérisé en ce que l'on fait réagir un composé de formule (I) dans laquelle Alk est un groupe triméthylène et R est un groupe éthyle avec soit un composé de formule (XIII) dans laquelle X est un groupe 3-chloro, soit un composé de formule (XIV) dans laquelle X est un groupe 3-chloro.

**13.** Procédé selon la revendication 8, pour la préparation de la N-[7-(éthoxycarbonylpentan-5-yloxy)-1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe pentaméthylène et R est un groupe éthyle avec soit un composé de formule (XIII) dans laquelle X est un groupe 3-chloro, soit un composé de formule (XIV) dans laquelle X est un groupe 3-chloro.

**14.** Procédé selon la revendication 8, pour la préparation de la N-[7-(2-éthoxycarbonylpropan-2-yloxy)-1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe isopropylidène et R est un groupe éthyle avec soit un composé de formule (XIII) dans laquelle X est un groupe 3-chloro, soit un composé de formule (XIV) dans laquelle X est un groupe 3-chloro.

**Revendications pour l'Etat contractant suivant : GR**

**1.** Un carboxyalkyl-éther de la 2-amino-7-hydroxytétraline de formule

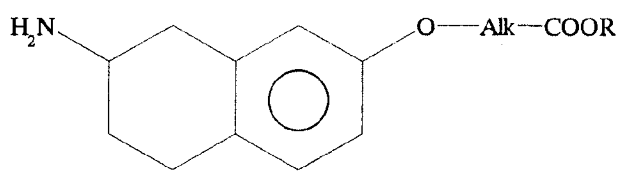

I

dans laquelle Alk représente un groupe alkylène de 3 à 5 atomes de carbone à chaîne droite ou ramifiée, et R représente l'hydrogène ou un groupe alkyle de 1 à 4 atomes de carbone, ou un de ses sels.

**2.** Composés selon la revendication 1, dans laquelle Alk représente
(a) un groupe

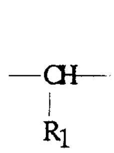 , 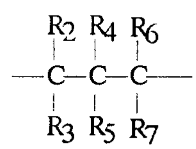 , 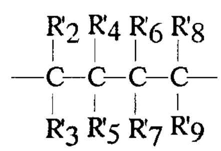 ,

ou $-(CH_2)_5-$
où

$R_1$ est un groupe éthyle, propyle ou butyle ;
$R_2$ à $R_7$ sont tous des hydrogènes ou un des radicaux $R_2$ à $R_7$ est un groupe méthyle ou éthyle et les autres sont des hydrogènes ou deux des radicaux $R_2$ à $R_7$ sont des groupes méthyle et les autres sont des hydrogènes ;
$R'_2$ et $R'_9$ sont tous des hydrogènes ou un des radicaux $R'_2$ à $R'_9$ est un groupe de méthyle et les autres sont des hydrogènes ;
(b) un groupe

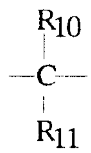

où
$R_{10}$ et $R_{11}$ représentent indépendamment un groupe méthyle ou éthyle ; ou $R_{11}$ est aussi le groupe propyle lorsque $R_{10}$ est méthyle ;
(c) un groupe

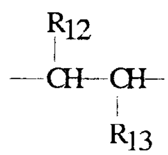

où l'un de $R_{12}$ et $R_{13}$ est un atome d'hydrogène et l'autre est un groupe méthyle, éthyle ou propyle, ou l'un de $R_{12}$ et $R_{13}$ est le groupe méthyle et l'autre est un groupe méthyle ou éthyle.

**3.** Composé selon la revendication 2, dans laquelle Alk représente un groupe

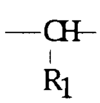 , 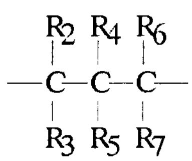 , 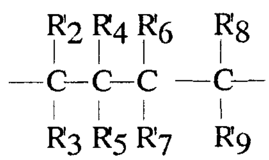 $-(CH_2)_5-$ ou 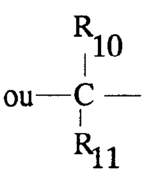

où $R_1$-$R_7$, $R_{10}$, $R_{11}$ et $R'_2$-$R'_9$ sont tels que définis dans la revendication 2.

**4.** Composé selon la revendication 3 qui est choisi parmi la 2-amino-7-(éthoxycarbonylpropan-3-yloxy)tétraline, la 2-amino-7-(éthoxycarbonylbutan-4-yloxy)tétraline, la 2-amino-7-(éthoxycarbonylpentan-5-yloxy)tétraline , la 2-amino-7(2-éthoxycarbonylpropan-2-yloxy)tétraline et leurs sels.

**5.** Un procédé pour la préparation d'un carboxyalkyl-éther de la 2-amino-7-hydroxytétraline de formule (I)

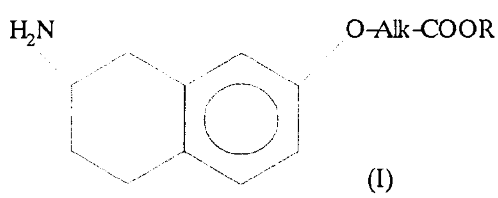

(I)

dans laquelle Alk et R sont tels que définis dans la revendication 1 ou d'un de ses sels, caractérisé en ce que
(A) on soumet une 2-amino-7-hydroxytétraline N-protégée de formule (II)

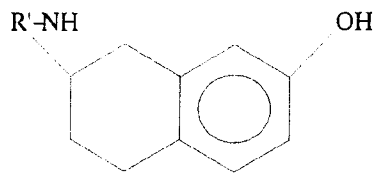

dans laquelle R' est un groupe protecteur du groupe amino qui peut être éliminé par hydrogénation catalytique ou par hydrolyse acide douce, à une réaction de carb(alk)oxyalkylation avec un composé de formule (IIIa)

$$Hal - Alk^1 - COOR \quad (IIIa)$$

dans laquelle R est tel que défini ci-dessus, Hal représente un atome de chlore, brome ou iode, et $Alk^1$ représente un groupe

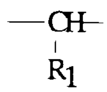 , 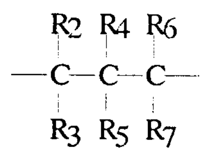 , 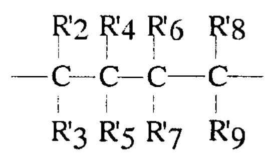 ,

ou $-(CH_2)_5-$
où
$R_1$ est un groupe éthyle, propyle ou butyle ;
$R_2$- à $R_7$ sont tous des hydrogènes ou un des radicaux $R_2$ à $R_7$ est un groupe méthyle ou éthyle et les autres sont des hydrogènes ou deux des radicaus $R_2$ à $R_7$ sont des groupes méthyle et les autres sont des hydrogènes ;
$R'_2$ à $R'_9$ sont tous des hydrogènes ou un des radicaux $R'_2$ à $R'_9$ est un groupe méthyle et les autres sont des hydrogènes ;
en présence d'un agent de condensation basique ;
ou avec un composé de formule (IIIb)

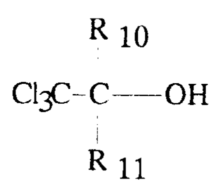
(IIIb)

dans laquelle
$R_{10}$ et $R_{11}$ représentent indépendamment un groupe méthyle ou éthyle, ou $R_{11}$ est aussi le groupe propyle lorsque $R_{10}$ est le groupe méthyle ; en présence d'une base forte, suivie éventuellement d'une réaction du composé ainsi obtenu avec du chlorure de thionyle dans un alcanol en $C_1$-$C_4$ convenablement choisi ;
ou avec un composé de formule (IIIc)

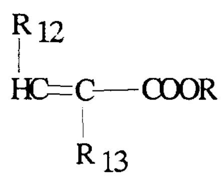
(IIIc)

dans laquelle R est tel que défini ci-dessus et l'un de $R_{12}$ et $R_{13}$ est l'hydrogène et l'autre est un groupe méthyle, éthyle ou propyle ou l'un de $R_{12}$ et $R_{13}$ est le groupe méthyle et l'autre est le groupe méthyle ou éthyle, éventuellement en présence de quantités catalytiques d'un hydroxyde d'ammonium quaternaire ; pour obtenir une 2-amino-7-hydroxytétraline N-protégée de formule (IV)

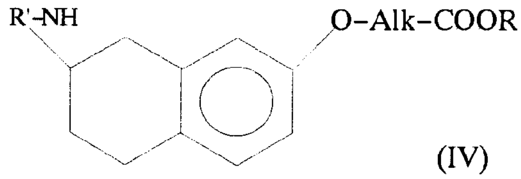

(IV)

dans laquelle Alk, R et R' sont tels que définis ci-dessus,
et
(B) on élimine le groupe N-protecteur par hydrogénation catalytique ou par hydrolyse acide douce et, après avoir éventuellement saponifié le groupe carbalcoxy inférieur en carboxy, on isole le composé de formule (I), sous forme de base libre ou de sel, et on le transforme éventuellement en l'un de ses sels.

6. Procédé selon la revendication 5 caractérisé en ce que le groupe N-protecteur R' est choisi parmi les groupes tert-butoxycarbonyle, benzyloxycarbonyle, et les groupes benzyle, benzhydryle et trityle, non substitués ou substitués par un groupe méthoxy sur un noyau phényle.

7. Un carboxyalkyl-éther d'une 2-anino-7-hydroxytétraline N-protégée de formule (IV)

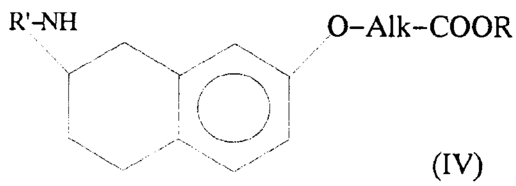

(IV)

dans laquelle R, Alk et R' sont tels que définis dans la revendication 5, ou un de ses sels.

8. Composé selon la revendication 7 où R' est tel que défini dans la revendication 6.

9. Composé selon la revendication 8, choisi parmi la 2-benzylamino-7-(éthoxycarbonylpropan-3-yloxy)tétraline, la 2-benzylamino-7-(éthoxycarbonylbutan-4-yloxy)tétraline, la 2-benzylamino-7-(éthoxycarbonylpentan-5-yloxy)tétraline, la 2-benzylamino-7-(2-éthoxycarbonylpropan-2-yloxy)tétraline, et leurs sels.

10. Procédé pour la préparation d'une phényléthanolaminotétraline de formule (XII)

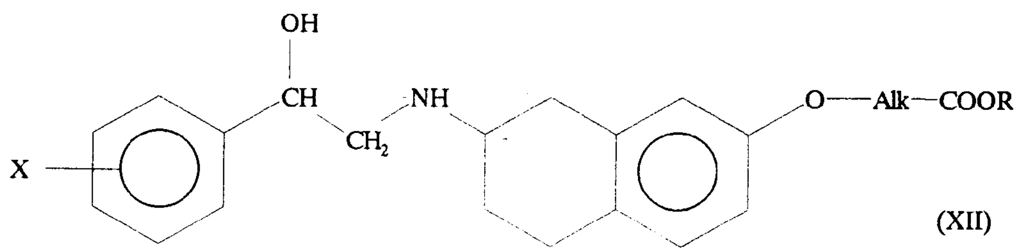

(XII)

dans laquelle
X représente l'hydrogène, un halogène, un groupe alkyle en $C_1$-$C_4$ ou le groupe trifluorométhyle,
R représente l'hydrogène ou un groupe alkyle en $C_1$-$C_4$ et Alk est tel que défini dans l'une quelconque des revendications 1 à 3, et ses sels pharmaceutiquement acceptables, caractérisé en ce qu'on fait réagir un composé de formule (I)

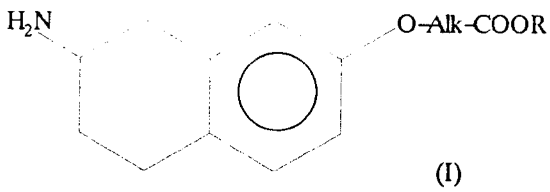

(I)

dans laquelle Alk représente un groupe alkylène de 3 à 5 atomes de carbone à chaîne droite ou ramifiée, et R est tel que défini ci-dessus, soit avec un époxyde de styrène de fomule (XIII)

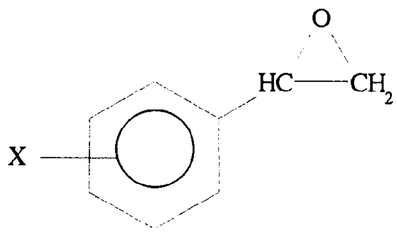

(XIII)

dans laquelle X est tel que défini ci-dessus,
soit avec un dérivé fonctionnel d'un acide mandélique de formule (XIV)

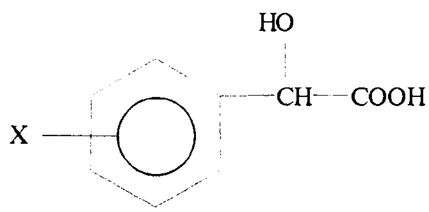

(XIV)

dans laquelle X est tel que défini ci-dessus et on réduit le groupe carbonyle amidique du mandélamide intermédiaire-ainsi obtenu de formule

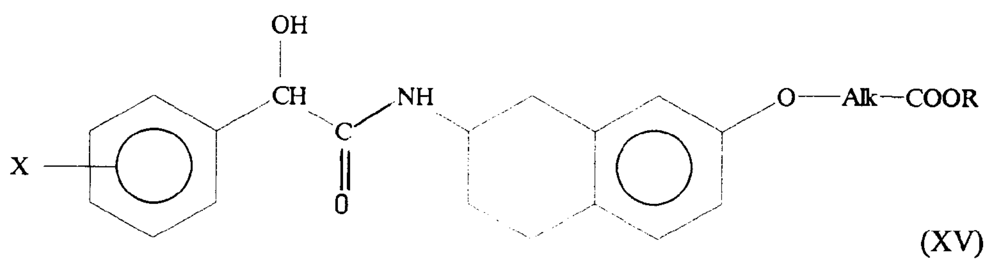

(XV)

dans laquelle X, Alk et R sont tels que définis ci-dessus, en groupe méthylène, et éventuellement on transforme le produit de formule (XII) ainsi obtenu dans un de ses sels pharmaceutiquement acceptables.

**11.** Procédé selon la revendication 10, caractérisé en ce que Alk représente un groupe

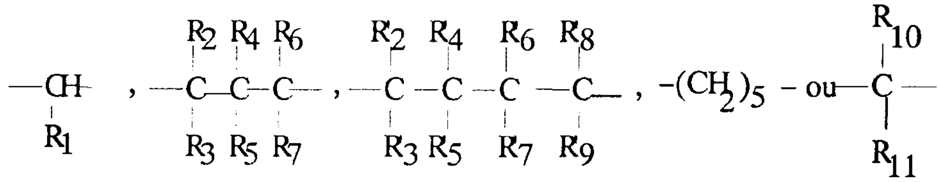

où

$R_1$ est un groupe éthyle, propyle ou butyle ;

$R_2$ à $R_7$ sont tous des hydrogènes ou un des radicaux $R_2$ à $R_7$ est un groupe méthyle ou éthyle et les autres sont des hydrogènes ou deux des radicaux $R_2$ à $R_7$ sont des groupes méthyle et les autres sont des hydrogènes ;

$R'_2$ à $R'_9$ sont tous des hydrogènes ou un des radicaux $R'_2$ à $R'_9$ est un groupe méthyle et les autres sont des hydrogènes ;

$R_{10}$ et $R_{11}$ représentent indépendamment un groupe méthyle ou éthyle, ou $R_{11}$ est aussi le groupe propyle lorsque $R_{10}$ est le groupe méthyle.

**12.** Procédé selon la revendication 11, caractérisé en ce que Alk représente un groupe

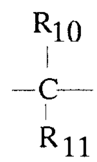

où $R_{10}$ et $R_{11}$ sont tels que définis dans la revendication 11.

**13.** Procédé selon la revendication 10, pour la préparation de la N-[7-(éthoxycarbonylbutan-4-yloxy)-1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe tétraméthylène et R est un groupe éthyle avec soit un composé de formule (XIII) dans laquelle X est un groupe 3-chloro, soit un composé de formule (XIV) dans laquelle X est un groupe 3-chloro.

**14.** Procédé selon la revendication 10, pour la préparation de la N-[7-(éthoxycarbonylpropan-3-yloxy)- 1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe triméthylène et R est un groupe éthyle avec soit un composé de formule (XIII) dans laquelle X est une groupe 3-chloro, soit un composé de formule (XIV) dans laquelle X est un groupe 3-chloro.

**15.** Procédé selon la revendication 10, pour la préparation de la N-[7-(éthoxycarbonylpentan-5-yloxy)- 1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe pentaméthylène et R est un groupe éthyle avec soit un composé de formule (XIII) dans laquelle X est un groupe 3-chloro, soit un composé de formule (XIV) dans laquelle X est un groupe 3-chloro.

**16.** Procédé selon la revendication 10, pour la préparation de la N-[7-(2-éthoxycarbonylpropan-2-yloxy)-1, 2, 3, 4-tétrahydronapht-2-yl]-2-hydroxy-2-(3-chlorophényl)éthanamine et de ses sels pharmaceutiquement acceptables, caractérisé en ce que l'on fait réagir un composé de formule (I) dans laquelle Alk est un groupe isopropylidène et R est un groupe éthyle avec soit un composé de formule (XIII) dans laquelle X est un groupe 3-chloro, soit un composé de formule (XIV) dans laquelle X est un groupe 3-chloro.

**17.** Procédé pour la préparation d'une composition pharmaceutique pour le traitement des troubles gastrointestinaux, caractérisé en ce qu'il consiste à mélanger un composé préparé selon l'une quelconque des revendications 10 à 16 avec un support pharmaceutiquement acceptable.

## Claims

### Claims for the following Contracting States : AT, BE, CH, DE, DK, FR, GB, LI, LU, NL, SE

**1.** A 2-amino-7-hydroxytetralin carboxyalkyl ether of formula (I)

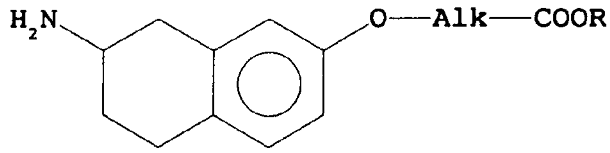

(I)

wherein Alk represents a straight or branched ($C_3$-$C_5$)-alkylene chain, and R represents hydrogen or ($C_1$-$C_4$)-alkyl or a salt thereof.

2. A compound according to claim 1, wherein Alk represents
   a) a group

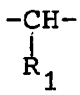

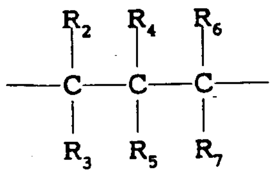

,

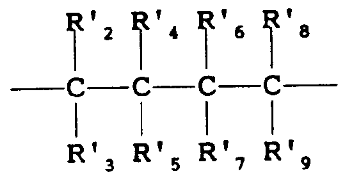

or $-(CH_2)_5-$
wherein
$R_1$ is an ethyl, propyl or butyl group,
$R_2$ to $R_7$ are all hydrogen atoms or one of $R_2$ to $R_7$ radicals is a methyl or ethyl group and the others are hydrogen atoms, or two of $R_2$ to $R_7$ are methyl groups and the others are hydrogen atoms;
$R'_2$ and $R'_9$ are all hydrogen atoms or one of $R'_2$ to $R'_9$ radicals is a methyl group and the others are hydrogen atoms;
b) a group

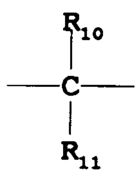

wherein
$R_{10}$ and $R_{11}$ are independently a methyl or ethyl group; or
$R_{11}$ is also the propyl group when $R_{10}$ is methyl;
c) a group

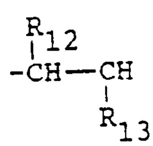

wherein
one of $R_{12}$ and $R_{13}$ is a hydrogen atom and the other is a methyl, ethyl or propyl group, or one of $R_{12}$ and $R_{13}$ is the methyl group and the other is a methyl or ethyl group.

3. Compound according to claim 2, wherein Alk represents a group:

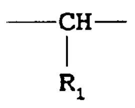 , 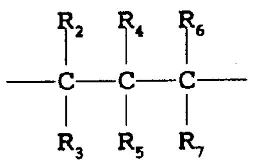 , 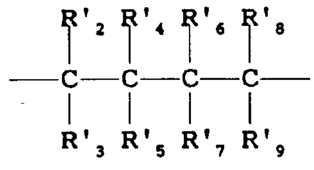 , —$(CH_2)_5$— or

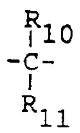

wherein $R_1$ to $R_{11}$ are as defined in claim 2.

4. Compound according to claim 3 which is selected from the group
2-amino-7-(ethoxycarbonylpropan-3-yloxy)tetralin,
2-amino-7-(ethoxycarbonylbutan-4-yloxy)tetralin,
2-amino-7-(ethoxycarbonylpentan-5-yloxy)tetralin,
2-amino-7-(2-ethoxycarbonylpropan-2-yloxy)tetralin, and
salts thereof.

5. A process for the preparation of a 2-amino-7-hydroxytetralin carboxyalkyl ether of formula (I)

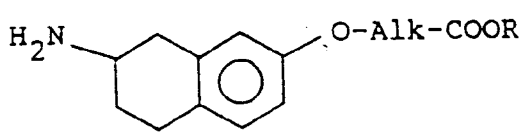

(I)

wherein Alk and R are as defined in claim 1, or a salt thereof, which comprises:
(A) submitting a N-protected 2-amino-7-hydroxytetralin of formula (II)

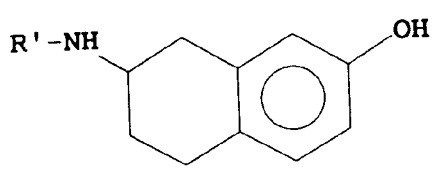

(II)

wherein
R' is an amino-protecting group which may suitably be removed by catalytic hydrogenation or mild acidic hydrolysis, to a carb(alk)oxyalkylation reaction with - a compound of formula (IIIa)

Hal - $Alk^1$ - COOR (IIIa)

wherein
R is as defined above,
Hal represents chloro, bromo or iodo, and $Alk_1$ represents a group

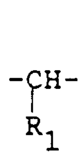 , 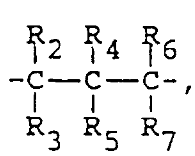 , 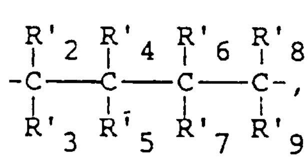 ,

or $-(CH_2)_5-$
wherein

$R_1$ may represent an ethyl, propyl or butyl group,

$R_2$ to $R_7$ are all hydrogen atoms or one of $R_2$ to $R_7$ is a methyl or ethyl group and the others are hydrogen atoms, or two of $R_2$ to $R_7$ radicals are methyl groups and the others are hydrogen atoms

$R'_2$ to $R'_9$ are all hydrogen atoms or one of $R'_2$ to $R'_9$ is a methyl group and the others are hydrogen atoms

in the presence of a basic condensation agent, or a compound of formula (IIIb)

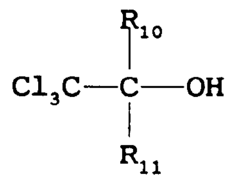

(IIIb)

wherein

$R_{10}$ and $R_{11}$ represents independently a methyl or ethyl group, or $R_{11}$ is also the propyl group, when $R_{10}$ is the methyl group; in the presence of a strong base, optionally followed by reaction of the obtained product with thionyl chloride in the suitably selected ($C_1$-$C_4$)alkanol, or with a compound of formula (IIIc)

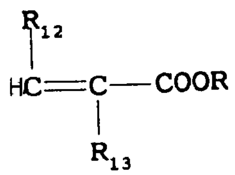

(IIIc)

wherein

R is as defined hereinabove and one of $R_{12}$ and $R_{13}$ is hydrogen and the other is a methyl, ethyl or propyl group, or one of $R_{12}$ and $R_{13}$ is the methyl group and the other is the methyl or ethyl group, optionally in the presence of catalytic amounts of a quaternary ammonium hydroxide,

to get a N-protected 2-amino-7-hydroxytetralin of formula (IV)

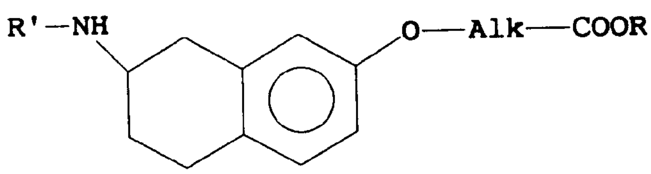

(IV)

wherein Alk, R and R' are as defined above, and

(B) removing the N-protecting group by catalytic hydrogenation or mild acidic hydrolysis and, after optional saponification of the lower carbalkoxy group to carboxy, isolating the compound of formula (I), as the free base or a salt thereof, and optionally converting it into one of its salts.

6. Process according to claim 5, characterized in that the N-protecting group R' is selected from the group consisting of tert-butoxycarbonyl, benzyloxycarbonyl, and the benzyl, benzhydryl, and trityl group, non-substituted or substituted by a methoxy group on a phenyl ring.

7. N-protected 2-amino-7-hydroxytetralin carboxyalkyl ether of formula (IV)

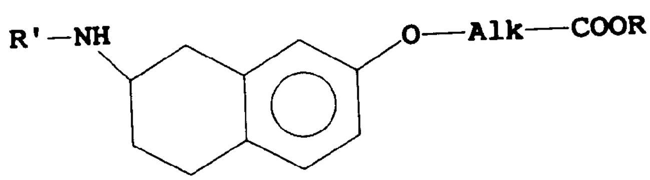

(IV)

wherein R, Alk, and R' are as defined in claim 5, or a salt thereof.

**8.** Compound according to claim 7 wherein R' is as defined in claim 6.

**9.** Compound according to claim 8, selected from the
2-benzylamino-7-(ethoxycarbonylpropan-3-yloxy)tetralin,
2-benzylamino-7-(ethoxycarbonylbutan-4-yloxy)tetralin,
2-benzylamino-7-(ethoxycarbonylpentan-5-yloxy)tetralin,
2-benzylamino-7-(2-ethoxycarbonylpropan-2-yloxy)tetralin,
and salts thereof.

**10.** A phenylethanolaminotetralin derivative of formula (XII)

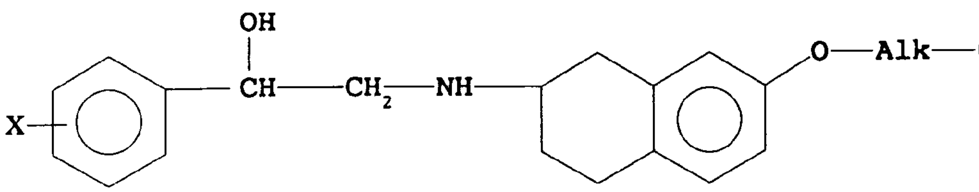

(XII)

wherein
X represents hydrogen, a halogen, a $(C_1-C_4)$alkyl group, or the trifluoromethyl group,
R represents hydrogen or a $(C_1-C_4)$alkyl group, and
Alk is as defined in any one of claims 1 to 3 and the pharmaceutically acceptable salts thereof.

**11.** A process for preparing a phenylethanolaminotetralin of claim 10, characterized in that it comprises reacting a compound of formula (I)

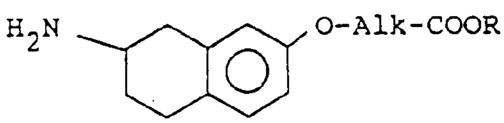

(I)

wherein Alk represents a straight or branched $(C_3-C_5)$-alkylene group, and R represents hydrogen or a $(C_1-C_4)$-alkyl group with a styrene oxide of formula (XIII)

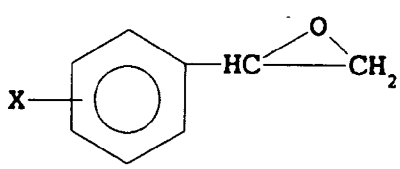

(XIII)

wherein X is as defined in claim 10, or with a functional derivative of a mandelic acid of formula (XIV)

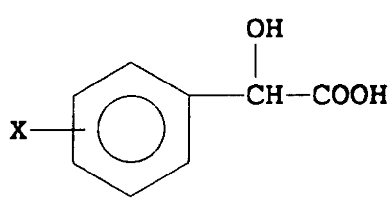

(XIV)

wherein X is as defined above, and reducing the amide carbonyl group of the thus obtained intermediate mandelamide derivative of formula (XV)

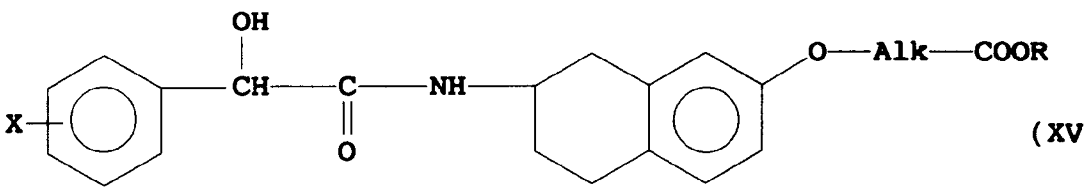

(XV)

wherein X, Alk and R are as defined above, into a methylene group, and optionally converting the obtained compounds (XII) into one of its pharmaceutically acceptable salts.

**12.** Compound according to claim 10, wherein Alk represents a group

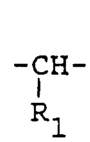 , 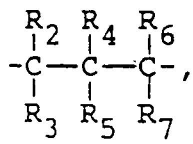 , 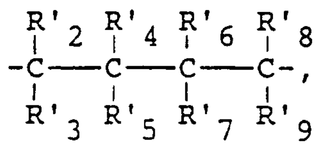 , $-(CH_2)_5-$, or

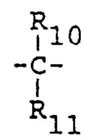

wherein

| | |
|---|---|
| $R_1$ | is an ethyl, propyl or butyl group, |
| $R_2$ to $R_7$ | are all hydrogen atoms or one of $R_2$ to $R_7$ is a methyl or ethyl group and the others are hydrogen atoms, or two of $R_2$ to $R_7$ are methyl groups and the others are hydrogen atoms; |
| $R'_2$ to $R'_9$ | are all hydrogen atoms or one of $R'_2$ to $R'_9$ is a methyl group and the others are hydrogen atoms; |
| $R_{10}$ and $R_{11}$ | represent independently a methyl or ethyl group, or $R_{11}$ is also the propyl group when $R_{10}$ is the methyl group. |

**13.** Compound according to claim 12, wherein Alk represents a group

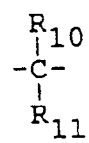

wherein $R_{10}$ and $R_{11}$ are as defined in claim 12.

**14.** Compound according to claim 10 which is N-[7-(ethoxycarbonylbutan-4-yloxy)-1,2,3,4-tetrahydronaphth-2-yl]-2-hydroxy-2-(3-chlorophenyl)ethanamine and the pharmaceutically acceptable salts thereof.

**15.** Compound according to claim 10 which is N-[7-(ethoxycarbonylpropan-3-yloxy)-1,2,3,4-tetrahydronaphth-2-yl] -2-hydroxy-2-(3-chlorophenyl)ethanamine and the pharmaceutically acceptable salts thereof.

**16.** Compound according to claim 10 which is N-[7-(ethoxycarbonylpentan-5-yloxy)-1,2,3,4-tetrahydronaphth-2-yl]-2-hydroxy-2-(3-chlorophenyl)ethanamine and the pharmaceutically acceptable salts thereof.

**17.** Compound according to claim 10 which is N-[7-(2-ethoxycarbonylpropan-2-yloxy)-1,2,3,4-tetrahydronaphth-2-yl]-2-hydroxy-2-(3-chlorophenyl)ethanamine and the pharmaceutically acceptable salts thereof.

**18.** Pharmaceutical composition for the treatment of gastrointestinal diseases, characterized in that it contains a compound of claim 10 as the active ingredient.

**Claims for the following Contracting State : ES**

**1.** Process for the preparation of 2-amino-7-hydroxytetralin carboxyalkyl ether of formula I

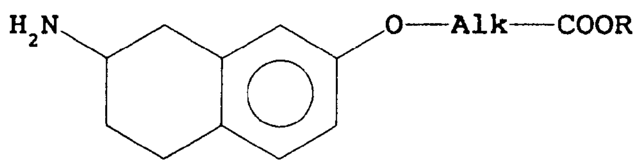

(I)

wherein Alk represents a straight or branched ($C_3$-$C_5$)-alkylene chain, and R represents hydrogen or ($C_1$-$C_4$)-alkyl or a salt thereof, characterized in that it comprises:
(A) submitting a N-protected 2-amino-7-hydroxytetralin of formula (II)

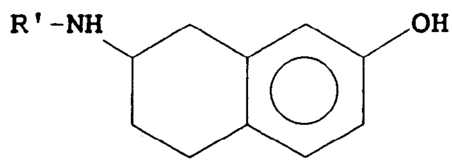

(II)

wherein R' is an amino-protecting group which may suitably be removed by catalytic hydrogenation or mild acidic hydrolysis, to a carb(alk)oxyalkylation reaction with a compound of formula (IIIa)

Hal - $Alk^1$ - COOR (IIIa)

wherein R is as defined above, Hal represents chloro, bromo or iodo, and $Alk_1$ represents

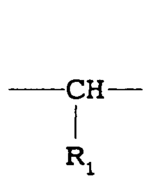 , 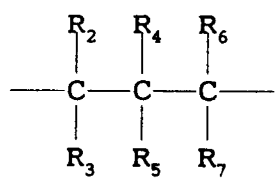 , 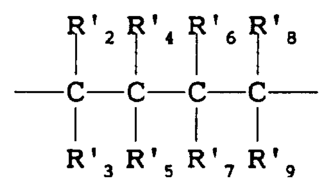

or $-(CH_2)_5-$ wherein
$R_1$ represents an ethyl, propyl or butyl group,
$R_2$ to $R_7$ are all hydrogen atoms or one of the radicals $R_2$ to $R_7$ is a methyl or ethyl group and the others are hydrogen atoms, or two of the radicals $R_2$ to $R_7$ are methyl groups and the others are hydrogen atoms;
$R'_2$ to $R'_9$ are all hydrogen atoms or one of $R'_2$ to $R'_9$ is a methyl group and the others are hydrogen atoms;
in the presence of a basic condensation agent, or with a compound of formula (IIIb)

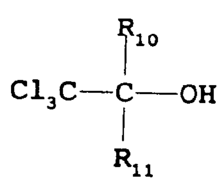

(IIIb)

wherein
$R_{10}$ and $R_{11}$ represents independently a methyl or ethyl group, or $R_{11}$ is also the propyl group when $R_{10}$ is the methyl group; in the presence of a strong base,

optionally followed by reaction of the obtained product with thionyl chloride in the suitably selected ($C_1$-$C_4$) alkanol, or with a compound of formula (IIIc)

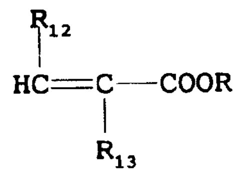

(IIIc)

wherein R is as defined above and one or $R_{12}$ and $R_{13}$ is hydrogen and the other is methyl, ethyl or propyl, or one of $R_{12}$ and $R_{13}$ is methyl and the other is a methyl or ethyl group, optionally in the presence of catalytic amounts of a quaternary ammonium hydroxide; to get a N-protected 2-amino-7-hydroxytetralin of formula (IV)

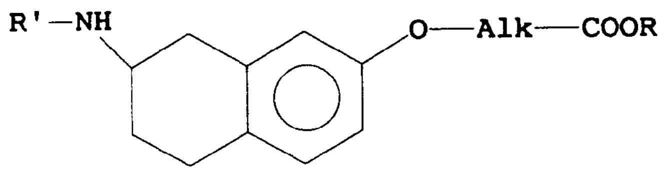

(IV)

wherein, Alk, R and R' are as defined above, and

(B) removing the N-protecting group by catalytic hydrogenation or mild acidic hydrolysis and, after optional saponification of the lower carbalkoxy group to carboxy, isolating the compound of formula (I), as the free base or a salt thereof, and optionally converting it into one of its salts.

2. Process according to claim 1, characterized in that the N-protecting group R' is selected from the group consisting of tert-butoxycarbonyl, benzyloxycarbonyl, and the benzyl, benzhydryl and trityl groups, non-substituted or substituted by a methoxy group on a phenyl ring.

3. Process according to one of claims 1 or 2, characterized in that Alk represents a group

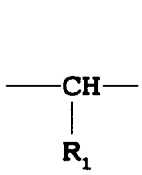 , 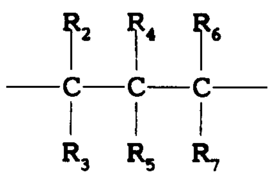 , 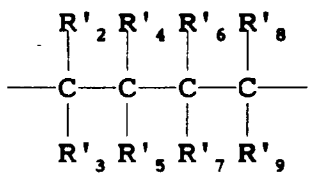 , $-(CH_2)_5-$

or 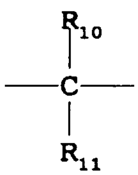

wherein $R_1$-$R_7$, $R_{10}$ and $R'_2$-$R'_9$ are as defined in claim 1.

4. Process according to claim 3, characterized in that it comprises reacting a compound of formula (II) wherein $R'_1$ is hydrogen with

* either a compound of formula (IIIa) wherein $Alk^1$ is a group

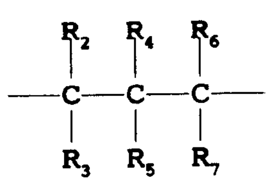 , 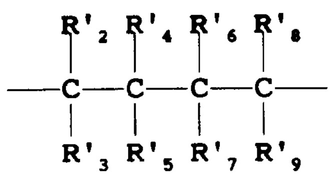

or $-(CH_2)_5-$
wherein $R_2$-$R_7$ and $R'_2$-$R'_9$ represent each a hydrogen atom and R an ethyl group;

* or a compound of formula (III b) wherein $R_{10}$ and $R_{11}$ are a methyl group, to form the following compounds:
2-amino-7-(ethoxycarbonylpropan-3-yloxy)tetralin,
2-amino-7-(ethoxycarbonylbutan-4-yloxy)tetralin,
2-amino-7-(ethoxycarbonylpentan-5-yloxy)tetralin,
2-amino-7-(2-ethoxycarbonylpropan-2-yloxy)tetralin, and
salts thereof.

**5.** Process for the preparation of an N-protected 2-amino-7-hydroxytetralin carboxyalkyl ether of formula (IV)

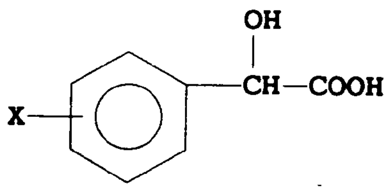

(XIV)

wherein R, Alk, and R' are as defined in claim 1, or a salt thereof, characterized in that it comprises:
(A) submitting a N-protected 2-amino-7-hydroxytetralin of formula (II)

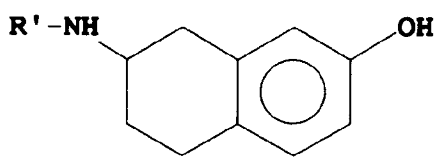

(II)

wherein
R' is an amino-protecting group which may suitably be removed by catalytic hydrogenation or mild acidic hydrolysis, to a carb(alk)oxyalkylation reaction with - a compound of formula (IIIa)

Hal - $Alk^1$ - COOR (IIIa)

wherein
R is as defined above,
Hal represents chloro, bromo or iodo, and $Alk_1$ represents

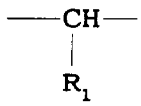 , 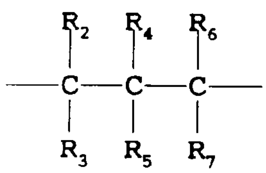 , 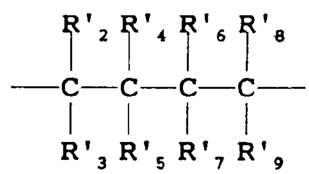

or $-(CH_2)_5-$
wherein
$R_1$ may represent an ethyl, propyl or butyl group,
$R_2$ to $R_7$ are all hydrogen atoms or one of $R_2$ to $R_7$ is a methyl or ethyl group and the others are hydrogen atoms, or two of $R_2$ to $R_7$ radicals are methyl groups and the others are hydrogen atoms,
$R'_2$ to $R'_9$ are all hydrogen atoms or one of $R'_2$ to $R'_9$ is a methyl group and the others are hydrogen atoms,
in the presence of a compound of formula (IIIb)

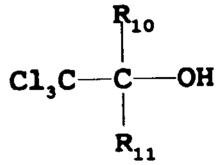

(IIIb)

wherein

$R_{10}$ and $R_{11}$ represents independently a methyl or ethyl group, or $R_{11}$ is also the propyl group, when $R_{10}$ is the methyl group; in the presence of a strong base, optionally followed by reaction of the obtained product with thionyl chloride in the suitably selected ($C_1$-$C_4$)alkanol, or with a compound of formula (IIIc)

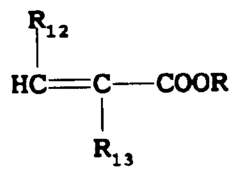

(IIIc)

wherein

R is as defined hereinabove and one of $R_{12}$ and $R_{13}$ is hydrogen and the other is a methyl, ethyl or propyl group, or one of $R_{12}$ and $R_{13}$ is the methyl group and the other is the methyl or ethyl group, optionally in the presence of catalytic amounts of a quaternary ammonium and optionally converting same into one of its salts.

6. Process according to claim 5, characterized in that R' is as defined in claim 2.

7. Process according to claim 6, characterized in that it comprises reacting a compound of formula (II) wherein R' is a benzyl group with
   * either a compound of formula (IIIa) wherein $Alk^1$ is a group

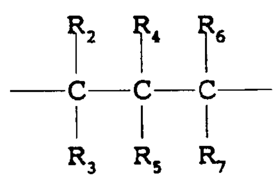

,

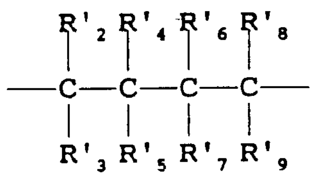

   or $-(CH_2)_5-$
   wherein $R_2$-$R_7$ and $R'_2$-$R'_9$ represent each a hydrogen atom and R an ethyl group;
   * or a compound of formula (III c) wherein $R_{12}$ is a methyl group, $R_{13}$ is hydrogen and R is an ethyl group to form the following compounds:
   2-benzylamino-7-(ethoxycarbonylpropan-3-yloxy)tetralin,
   2-benzylamino-7-(ethoxycarbonylbutan-4-yloxy)tetralin,
   2-benzylamino-7-(ethoxycarbonylpentan-5-yloxy)tetralin,
   2-benzylamino-7-(ethoxycarbonylpropan-2-yloxy)tetralin,
   and salts thereof.

8. Process for the preparation of a phenylethanolaminotetralin derivative of formula (XII)

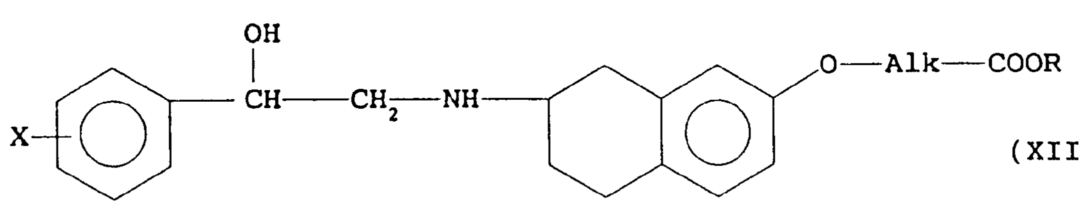

(XII)

wherein
X represents hydrogen, halogen, ($C_1$-$C_4$) alkyl, or trifluoromethyl,
R represents hydrogen or a ($C_1$-$C_4$) alkyl group, and Alk is as defined in any of claims 1 or 3, and the pharmaceutically acceptable salts thereof, characterized in that it comprises reacting a compound of formula (I)

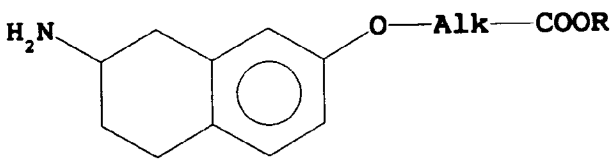

(I)

wherein Alk represents a straight or branched ($C_3$-$C_5$)-alkylene chain, and R represents hydrogen or ($C_1$-$C_4$)-alkyl

* either with a styrene oxide of formula (XIII)

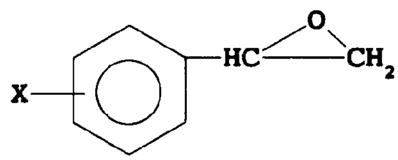

(XIII)

wherein X is as defined above,

* or with a functional derivative of a mandelic acid of formula (XIV)

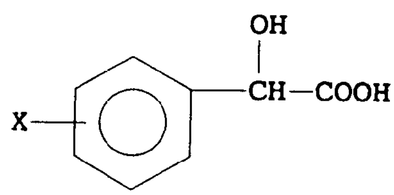

(XIV)

wherein X is as defined above, and reducing the amide carbonyl group of the thus obtained intermediate mandelamide derivative of formula

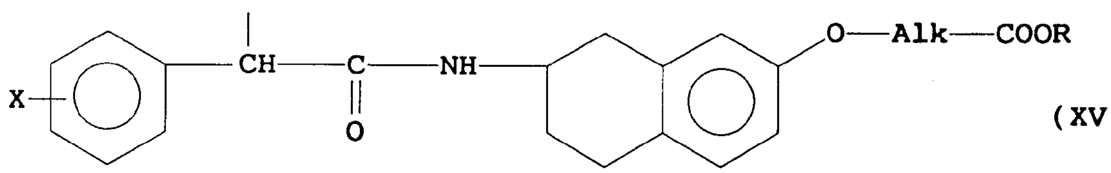

(XV)

wherein X, R, and Alk are as defined above, into a methylene group,
and optionally converting the obtained compounds (XII) into the corresponding'pharmaceutically acceptable salts.

9. Process according to claim 8, characterized in that Alk represents group

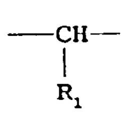 , 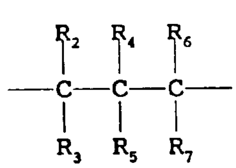 , 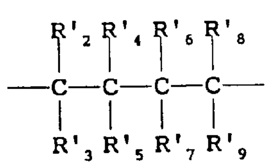 , $-(CH_2)_5-$ or 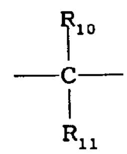

wherein
$R_1$ may represent ethyl, propyl or butyl,

$R_2$ to $R_7$ are all hydrogen atoms or one of $R_2$ to $R_7$ is a methyl or ethyl group and the others are hydrogen atoms, or two of $R_2$ to $R_7$ radicals are methyl groups and the others are hydrogen atoms;
$R'_2$ to $R'_9$ are all hydrogen atoms or one of $R'_2$ to $R'_9$ is a methyl group and the others are hydrogen atoms;
$R_{10}$ and $R_{11}$ are independently a methyl or ethyl group, or $R_{11}$ is also the propyl group when $R_{10}$ is the methyl group.

**10.** Process according to claim 9, characterized in that Alk represents a group

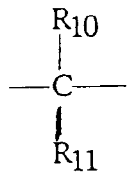

wherein $R_{10}$ and $R_{11}$ are as defined in claim 9.

**11.** Process according to claim 8, for the preparation of N-[7-ethoxycarbonylbutan-4-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I) wherein Alk is a tetramethylene group and R is an ethyl group with either a compound of formula (XIII) wherein X is a 3-chloro group, or a compound of formula (XIV) wherein X is a 3-chloro group.

**12.** Process according to claim 8 for the preparation of N-[7-ethoxycarbonylpropan-3-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I) wherein Alk is a trimethylene group and R is an ethyl group with either a compound of formula (XIII) wherein X is a 3-chloro group, or a compound of formula (XIV) wherein X is a 3-chloro group.

**13.** Process according to claim 8, for the preparation of N-[7-(ethoxycarbonylpentan-5-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I) wherein Alk is a pentamethylene group and R is an ethyl group with either a compound of formula (XIII) wherein X is a 3-chloro group, or a compound of formula (XIV) wherein X is a 3-chloro group.

**14.** Process according to claim 8, for the preparation of N-[7-(ethoxycarbonylpropan-2-yloxy)-1,2,3,4-tetrahydro-naphth-2-yl]-2-hydroxy-2-(3-chlorophenyl)ethanamine and the pharmaceutically acceptable salts thereof, characterized in that it comprises reacting a compound of formula (I), wherein Alk is an isopropylidene group and R is an ethyl group with either a compound of formula (XIII) wherein X is a 3-chloro group, or a compound of formula XIV wherein X is a 3-chloro group.

**Claims for the following Contracting State : GR**

**1.** A 2-amino-7-hydroxytetralin carboxyalkyl ether of formula (I)

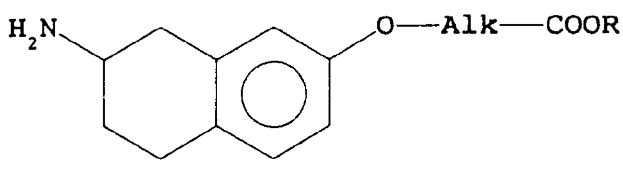

(I)

wherein Alk represents a straight or branched ($C_3$-$C_5$)-alkylene chain, and R represents hydrogen or ($C_1$-$C_4$)-alkyl or a salt thereof.

**2.** Compounds according to claim 1 wherein Alk represents
a) a group

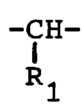 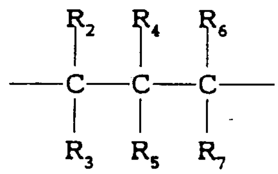 , 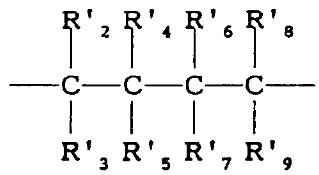

or $-(CH_2)_5-$

wherein

$R_1$ is ethyl, propyl or butyl,

$R_2$ to $R_7$ are all hydrogen atoms or one of $R_2$ to $R_7$ is a methyl or ethyl group and the others are hydrogen atoms, or two of $R_2$ to $R_7$ are methyl groups and the others are hydrogen atoms ;

$R'_2$ and $R'_9$ are all hydrogen atoms or one of $R'_2$ to $R'_9$ is a methyl group and the others are hydrogen atoms;

b) a group

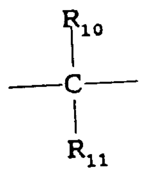

wherein

$R_{10}$ and $R_{11}$ are independently methyl or ethyl or

$R_{11}$ is also propyl when $R_{10}$ is methyl;

c) a group

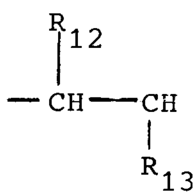

wherein

one of $R_{12}$ and $R_{13}$ is a hydrogen atom and the other is a methyl, ethyl or propyl group, or one of $R_{12}$ and $R_{13}$ is the methyl group and the other is a methyl or ethyl group.

3. Compound according to claim 2 wherein Alk represents

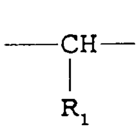 , 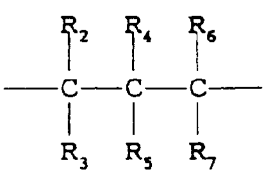 , 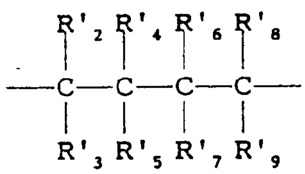 , $-CH_2)_5-$ or 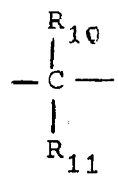

wherein $R_1$-$R_7$, $R_{10}$, $R_{11}$ and $R'_2$-$R'_9$ are as defined in claim 2.

4. Compound according to claim 3 which is selected from the group
2-amino-7-(ethoxycarbonyl-propan-3-yloxy)tetralin,
2-amino-7-(ethoxycarbonylbutan-4-yloxy)tetralin,
2-amino-7-(ethoxycarbonylpentan-5-yloxy)tetralin,
2-amino-7-(2-ethoxycarbonylpropan-2-yloxy)tetralin, and

salts thereof.

5. A process for the preparation of a 2-amino-7-hydroxytetralin carboxyalkyl ether of formula (I)

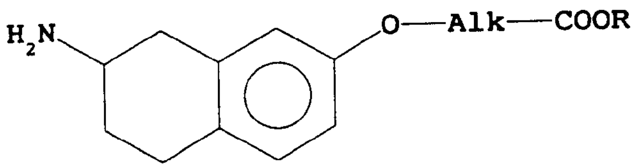

(I)

wherein Alk and R are as defined in claim 1, or a salt thereof, characterized in that it comprises:

(A) submitting a N-protected 2-amino-7-hydroxytetralin of formula (II)

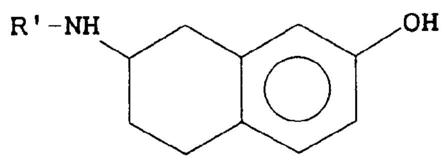

wherein

R' is an amino-protecting group which may suitably be removed by catalytic hydrogenation or mild acidic hydrolysis, to a carb(alk)oxyalkylation reaction with - a compound of formula (IIIa)

Hal - $Alk^1$ - COOR (IIIa)

wherein

R is as defined above,

Hal represents chloro, bromo or iodo, and $Alk_1$ represents

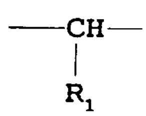 , 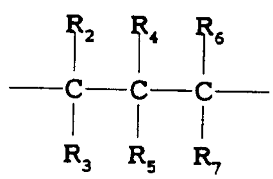 , 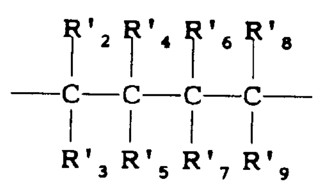

or-$(CH_2)_5$- wherein

$R_1$ may represent ethyl, propyl or butyl,

$R_2$ to $R_7$ are all hydrogen atoms or one of $R_2$ to $R_7$ is a methyl or ethyl group and the others are hydrogen atoms, or two of $R_2$ to $R_7$ are methyl groups and the others are hydrogen atoms;

$R'_2$ to $R'_9$ are all hydrogen atoms or one of $R'_2$ to $R'_9$ is a methyl group and the others are hydrogen atoms in the presence of a compound of formula (IIIb)

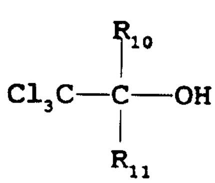

(IIIb)

wherein

$R_{10}$ and $R_{11}$ independently represent a methyl or ethyl group, or $R_{11}$ is also the propyl group, when $R_{10}$ is the methyl group; in the presence of a strong base, optionally followed by reaction of the obtained product with thionyl chloride in the suitably selected ($C_1$-$C_4$)alkanol, or with a compound of formula (IIIc)

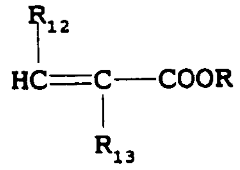

(IIIc)

wherein

R is as defined above and one of $R_{12}$ and $R_{13}$ is hydrogen and the other is a methyl, ethyl or propyl group, or one of $R_{12}$ and $R_{13}$ is the methyl group and the other is a methyl or ethyl group, optionally in the presence of catalytic amounts of a quaternary ammonium hydroxide,

to get a N-protected 2-amino-7-hydroxytetralin of formula (IV)

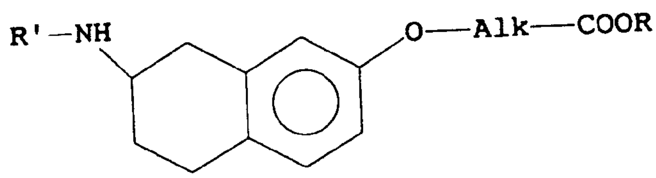

(IV)

wherein Alk, R and R' are as defined above, and

(B) removing the N-protecting group by catalytic hydrogenation or mild acidic hydrolysis and, after optional saponification of the lower carbalkoxy group to carboxy, isolating the compound of formula (I), as the free base or a salt thereof, and optionally converting it into one of its salts.

6. Process according to claim 5, characterized in that the N-protecting group is selected from the group consisting of tert-butoxycarbonyl, benzyloxycarbonyl, and the benzyl, benzhydryl and trityl groups non-substituted or substituted by a methoxy group on a phenyl ring.

7. An N-protected 2-amino-7-hydroxytetralin carboxyalkyl ether of formula (IV)

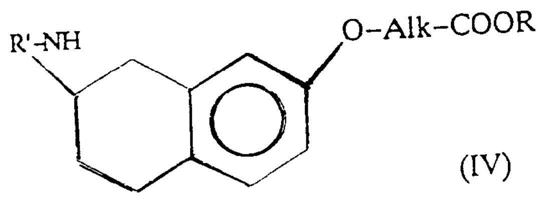

(IV)

wherein R, Alk, and R' are as defined in claim 5, or a salt thereof.

8. Compound according to claim 7 wherein R' is as defined in claim 6.

9. Compound according to claim 8, selected from the
2-benzylamino-7-(ethoxycarbonylpropan-3-yloxy)tetralin,
2-benzylamino-7-(ethoxycarbonylbutan-4-yloxy)tetralin,
2-benzylamino-7-(ethoxycarbonylpentan-5-yloxy)tetralin,
2-benzylamino-7-(2-ethoxycarbonylpropan-2-yloxy)tetralin,
and salts thereof.

10. Process for the preparation of a phenylethanolaminotetralin derivative of formula (XII)

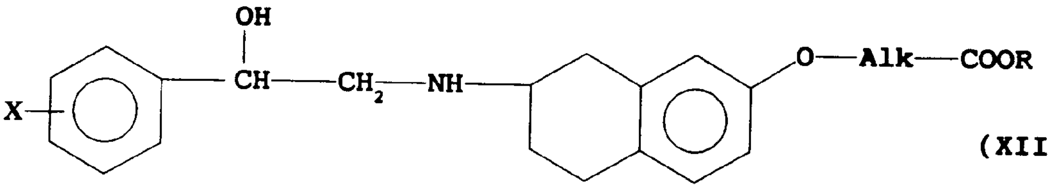

(XII)

wherein

X represents hydrogen, a halogen, a ($C_1$-$C_4$)alkyl group, or the trifluoromethyl group,

R represents hydrogen or a ($C_1$-$C_4$)alkyl group, and

Alk is as defined in any one of claims 1 to 3, and the pharmaceutically acceptable salts thereof, characterized in that it comprises reacting a compound of formula (I)

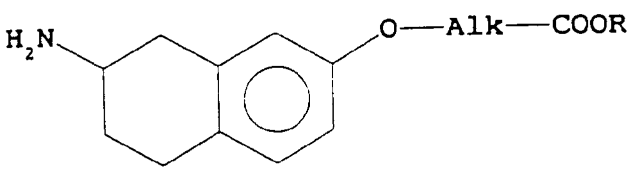

(I)

wherein Alk represents a straight or branched ($C_3$-$C_5$)-alkylene chain, and R is as above defined,

- either with a styrene oxide of formula (XIII)

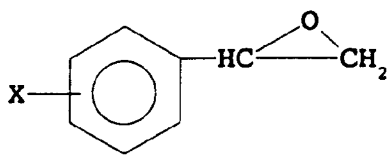

(XIII)

wherein X is as defined above,

- or with a functional derivative of a mandelic acid of formula (XIV)

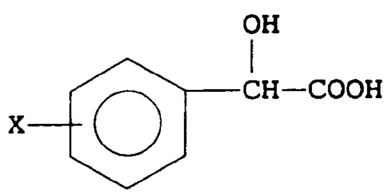

(XIV)

wherein X is as defined above, and reducing the amide carbonyl group of the thus obtained intermediate mandelamide derivative of formula

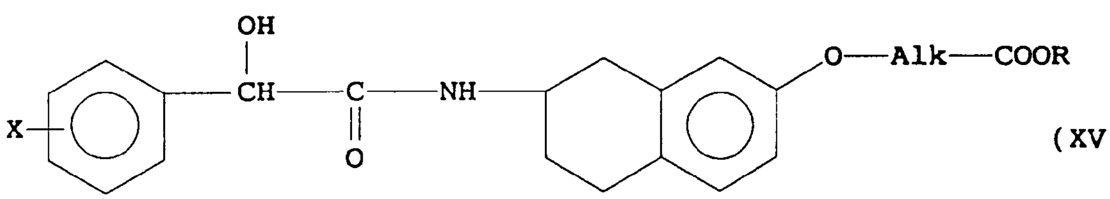

(XV)

wherein X, R, and Alk are as defined above, into a methylene group and optionally converting the obtained compounds (XII) into the corresponding pharmaceutically acceptable salts.

**11.** Process according to claim 10, wherein Alk represents

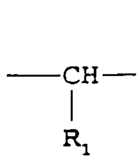 , 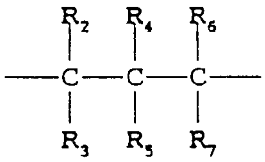 , 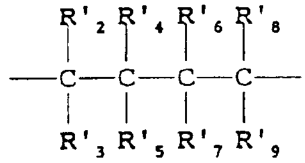 , $-CH_2)_5-$ or 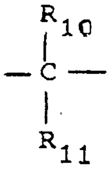

wherein

| | |
|---|---|
| $R_1$ | is an ethyl, propyl or butyl group, |
| $R_2$ to $R_7$ | are all hydrogen atoms or one of $R_2$ to $R_7$ is a methyl or ethyl group and the others are hydrogen atoms, or two if $R_2$ to $R_7$ are methyl groups and the others are hydrogen atoms |
| $R'_2$ to $R'_9$ | are all hydrogen atoms or one of $R'_2$ to $R'_9$ is a methyl group and the others are hydrogen atoms |
| $R_{10}$ and $R_{11}$ | represents independently a methyl or ethyl group, or $R_{11}$ is alo the propyl group when $R_{10}$ is the methyl group. |

**12.** Compound according to claim 11, wherein Alk represents a group

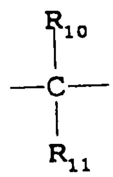

wherein $R_{10}$ and $R_{11}$ are as defined in claim 11.

**13.** Process according to claim 10, for the preparation of N-[7-ethoxycarbonylbutan-4-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I) wherein Alk is a tetramethylene group and R is an ethyl group with either a compound of formula (XIII) wherein X is a 3-chloro group, or a compound of formula (XIV) wherein X is a 3-chloro group.

**14.** Process according to claim 10 for the preparation of N-[7-ethoxycarbonylpropan-3-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I) wherein Alk is a trimethylene group and R is an ethyl group with either a compound of formula (XIII) wherein X is a 3-chloro group, or a compound of formula (XIV) wherein X is a 3-chloro group.

**15.** Process according to claim 8, for the preparation of N-[7-(ethoxycarbonylpentan-5-yloxy)-1,2,3,4-tetrahydronaphth-2-yl]-2-hydroxy-2-(3-chlorophenyl)ethanamine and the pharmaceutically acceptable salts thereof, characterized in that reacting a compound of formula (I) wherein Alk is a pentamethylene group and R is an ethyl group with either a compound of formula (XIII) wherein X is a 3-chloro group, or a compound of formula (XIV) wherein X is a 3-chloro group.

**16.** Process according to claim 8, for the preparation of N-[7-(ethoxycarbonylpropan-2-yloxy)-1,2,3,4-tetrahydronaphth-2-yl]-2-hydroxy-2-(3-chlorophenyl)ethanamine and the pharmaceutically acceptable salts thereof, characterized in that it comprises reacting a compound of formula (I), wherein Alk is an isopropylidene group and R is an ethyl group with either a compound of formula (XIII) wherein X is a 3-chloro group, or a compound of formula (IXV) wherein X is a 3-chloro group.

**17.** Process for the preparation of a pharmaceutical composition for the treatment of gastrointestinal diseases, characterized in that it consists in mixing a compound prepared according to any one of claims 10 to 16 with a pharmaceutically acceptable base.

## Patentansprüche

### Patentansprüche für folgende Vertragsstaaten : AT, BE, CH, DE, DK, FR, GB, LI, LU, NL, SE

1. Carboxyalkylether des 2-Amino-7-hydroxytetralins der Formel I,

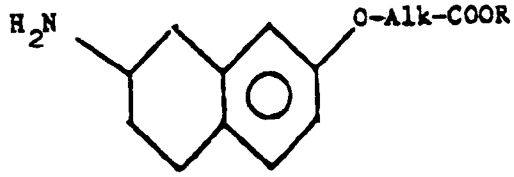

I,

in der bedeuten:

Alk geradkettiges oder verzweigtes $C_{3-5}$-Alkylen und

R Wasserstoff oder $C_{1-4}$-Alkyl,

sowie eines seiner Salze.

2. Verbindung nach Anspruch 1, in der Alk bedeutet:

(a) eine Gruppe

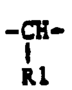 , 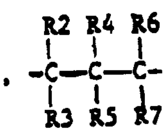 , 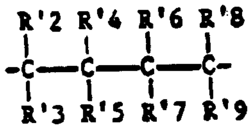

oder $-(CH_2)_5-$ ,

wobei bedeuten:

R1 Ethyl, Propyl oder Butyl;

R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff;

(b) eine Gruppe

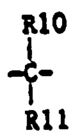 ,

wobei bedeuten:

R10 und R11 unabhängig Methyl oder Ethyl, wobei R11 auch Propyl bedeuten kann, wenn R10 Methyl ist;

(c) eine Gruppe

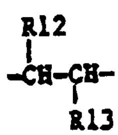 ,

wobei bedeuten:

eine der Gruppen R12 und R13 Wasserstoff und die andere Methyl, Ethyl oder Propyl oder eine der Gruppen R12 und R13 Methyl und die andere Methyl oder Ethyl.

3. Verbindung nach Anspruch 2, wobei Alk eine Gruppe

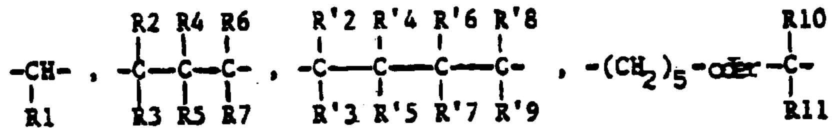

bedeutet, bei denen R1 bis R11 die in Anspruch 2 definierte Bedeutung besitzen.

4. Verbindung nach Anspruch 3, die ausgewählt ist unter
2-Amino-7-(ethoxycarbonylpropan-3-yloxy)-tetralin,
2-Amino-7-(ethoxycarbonylbutan-4-yloxy)-tetralin,
2-Amino-7-(ethoxycarbonylpentan-5-yloxy)-tetralin, und
2-Amino-7-(2-ethoxycarbonylpropan-2-yloxy)-tetralin sowie deren Salzen.

5. Verfahren zur Herstellung eines Carboxyalkylethers des 2-Amino-7-hydroxytetralins der Formel I,

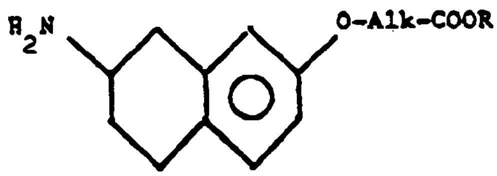

(I),

in der Alk und R die in Anspruch 1 definierte Bedeutung besitzen,
sowie eines seiner Salze,
gekennzeichnet durch
(A) Carb(alk)oxyalkylierung eines N-geschützten 2-Amino-7-hydroxytetralins der Formel II,

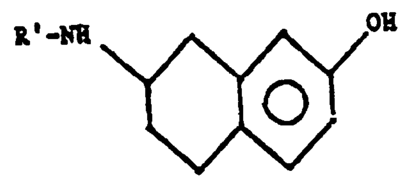

(II),

in der R' eine Aminoschutzgruppe bedeutet, die durch katalytische Hydrierung oder saure Hydrolyse unter milden Bedingungen abgespalten werden kann, entweder mit einer Verbindung der Formel IIIa,

$$\text{Hal - Alk}^1\text{ - COOR} \quad \text{(IIIa)},$$

in der bedeuten:
R dasselbe wie oben definiert,
Hal ein Atom Chlor, Brom oder Jod
und
Alk[1] eine Gruppe

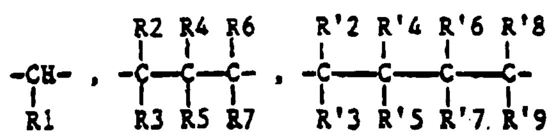

oder $(CH_2)_5$-,
wobei bedeuten:
R1 Ethyl, Propyl oder Butyl;
R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;
R'2 bis R'9 sämtlich Wasserstoff oder eine der Gruppen R'2 bis R'9 Methyl und die übrigen Wasserstoff,
in Gegenwart eines basischen Kondensationsmittels;
oder mit einer Verbindung der Formel IIIb,

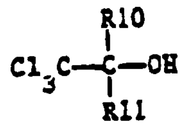 (IIIb),

worin bedeuten:

R10 und R11 unabhängig Methyl oder Ethyl, wobei R11 auch Propyl bedeuten kann, wenn R10 Methyl ist,

in Gegenwart einer starken Base;

sowie gegebenenfalls anschließende Umsetzung der so erhaltenen Verbindung mit Thionylchlorid in einem geeignet ausgewählten $C_{1-4}$-Alkanol;

oder mit einer Verbindung der Formel IIIc,

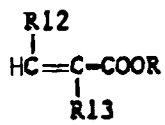 (IIIc),

worin bedeuten:

R dasselbe wie oben definiert

und

eine der Gruppen R12 und R13 Wasserstoff und die andere Methyl, Ethyl oder Propyl oder eine der Gruppen R12 und R13 Methyl und die andere Methyl oder Ethyl,

gegebenenfalls in Gegenwart katalytischer Mengen eines quaternären Ammoniumhydroxids, unter Erhalt eines N-geschützten 2-Amino-7-hydroxytetralins der Formel IV,

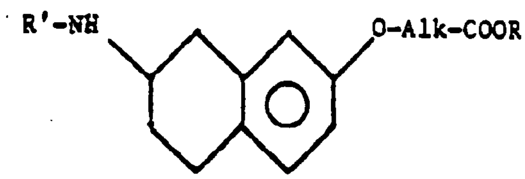 (IV),

in der Alk, R und R' die obige Bedeutung besitzen, und

(B) Abspaltung der N-Schutzgruppe durch katalytische Hydrierung oder saure Hydrolyse unter milden Bedingungen sowie, gegebenenfalls nach Verseifung der niederen Carbalkoxygruppe zu einer Carboxygruppe, Isolierung der Verbindung der Formel I in Form der freien Base oder eines Salzes und gegebenenfalls Umwandlung in eines ihrer Salze.

**6.** Verfahren nach Anspruch 5, dadurch gekennzeichnet, daß die N-Schutzgruppe R' unter Tertiärbutoxycarbonyl, Benzyloxycarbonyl, Benzyl, Benzhydryl und Trityl, die am Phenylkern nichtsubstituiert oder mit einer Methoxygruppe substituiert sein können, ausgewählt wird.

**7.** Carboxyalkylether eines N-geschützten 2-Amino-7-hydroxytetralins der Formel IV,

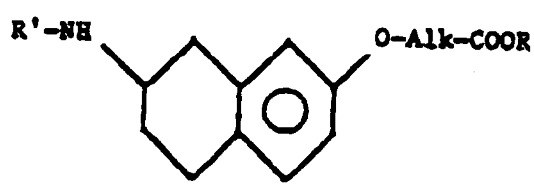 (IV),

in der R, Alk und R' die in Anspruch 5 definierte Bedeutung besitzen, oder eines seiner Salze.

**8.** Verbindung nach Anspruch 7, wobei R' die in Anspruch 6 definierte Bedeutung besitzt.

**9.** Verbindung nach Anspruch 8, die ausgewählt ist unter
2-Benzylamino-7-(ethoxycarbonylpropan-3-yloxy)-tetralin,
2-Benzylamino-7-(ethoxycarbonylbutan-4-yloxy)-tetralin,
2-Benzylamino-7-(ethoxycarbonylpentan-5-yloxy)-tetralin und
2-Benzylamino-7-(2-ethoxycarbonylpropan-2-yloxy)-tetralin,
sowie deren Salzen.

**10.** Phenylethanolaminotetralin der Formel (XII),

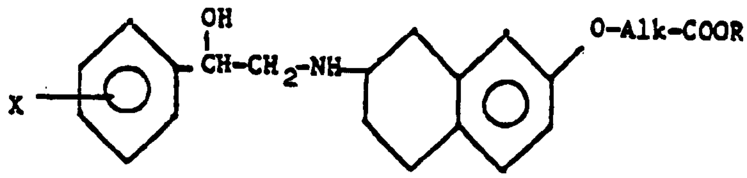

(XII),

in der bedeuten:
X Wasserstoff, Halegen, $C_{1-4}$-Alkyl oder Trifluormethyl,
R Wasserstoff oder $C_{1-4}$-Alkyl
und
Alk dasselbe wie in einem der Ansprüche 1 bis 3 definiert,
sowie seine pharmazeutisch akzeptablen Salze.

**11.** Verfahren zur Herstellung eines Phenylethanolaminotetralins nach Anspruch 10,
gekennzeichnet durch
Umsetzung einer Verbindung der Formel I,

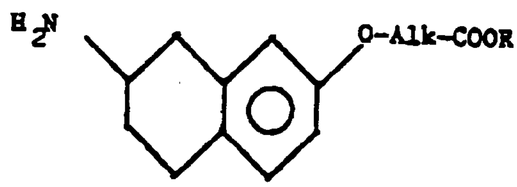

(I),

in der bedeuten:
Alkgeradkettiges oder verzweigtes $C_{3-5}$-Alkylen und
R Wasserstoff oder $C_{1-4}$-Alkyl,
mit einem Styrolepoxid der Formel XIII,

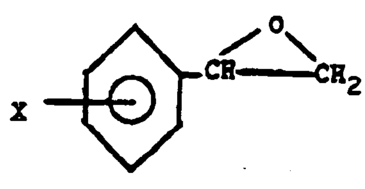

(XIII),

in der X die in Anspruch 10 definierte Bedeutung besitzt,
oder Umsetzung mit einem funktionellen Derivat einer Mandelsäure der Formel XIV,

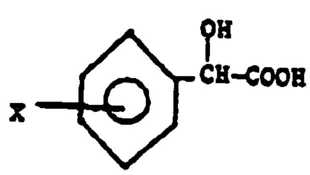

(XIV),

in der X die oben definierte Bedeutung besitzt, und
Reduktion der Carbonsäureamidgrupge des so als Zwischenprodukt erhaltenen Mandelamids der Formel XV,

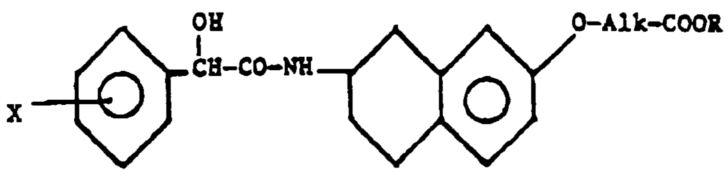

(XV),

in der X, Alk und R die oben definierte Bedeutung besitzen,
zu einer Methylengruppe
sowie gegebenenfalls Umwandlung der so erhaltenen Verbindung der Formel XII in eines ihrer pharmazeutisch akzeptablen Salze.

**12.** Verbindung nach Anspruch 10, wobei Alk eine Gruppe

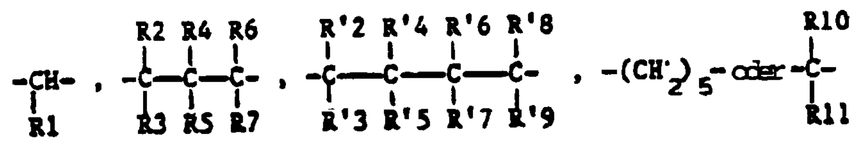

darstellt, wobei bedeuten:
R1 Ethyl, Propyl oder Butyl;
R2 bis R7 sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;
R'2 bis R'9 sämtlich Wasserstoff oder eine der Gruppen R'2 bis R'9 Methyl und die übrigen Wasserstoff;
R10 und R11 unabhängig Methyl oder Ethyl, wobei R1 auch Propyl sein kann, wenn R10 Methyl ist.

**13.** Verbindung nach Anspruch 12, wobei Alk eine Gruppe

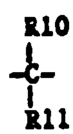

darstellt, bei der R10 und R11 die in Anspruch 12 definierte Bedeutung besitzen.

**14.** N-[7-(ethoxycarbonylbutan-4-yloxy)-1,2,3,4-tetrahydronaphth-2-yl]-2-hydroxy-1-(3-chlorphenyl)-ethanamin und seine pharmazeutisch akzeptablen Salze als Verbindung nach Anspruch 10.

**15.** N-[7-(ethoxycarbonylpropan-3-yloxy)-1,2,3,4-tetrahydronaphth-2-yl]-2-hydroxy-2-(3-chlorphenyl)-ethanamin und seine pharmazeutisch akzeptablen Salze als Verbindung nach Anspruch 10.

**16.** N-[7-(ethoxycarbonylpentan-5-yloxy)-1,2,3,4-tetrahydronaphth-2-yl]-2-hydroxy-2-(3-chlorphenyl)-ethanamin und seine pharmazeutisch akzeptablen Salze als Verbindung nach Anspruch 10.

**17.** N-[7-(2-ethoxycarbonylpropan-2-yloxy)-1,2,3,4-tetrahydronaphth-2-yl]-2-hydroxy-2-(3-chlorphenyl)-ethanamin und seine pharmazeutisch akzeptablen Salze als Verbindung nach Anspruch 10.

**18.** Pharmazeutische Zusammensetzung zur Behandlung gastrointestinaler Störungen, dadurch gekennzeichnet, daß sie als Wirkstoff eine verbindung nach Anspruch 10 enthält.

**Patentansprüche für folgenden Vertragsstaat : ES**

1. Verfahren zur Herstellung eines Carboxyalkylethers des 2-Amino-7-hydroxytetralins der Formel I

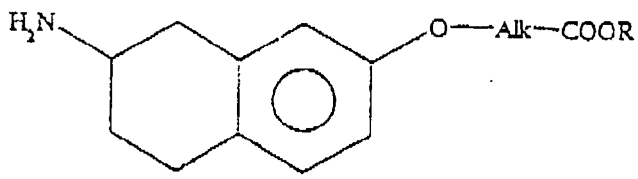

(I),

in der bedeuten:

Alk geradkettiges oder verzweigtes $C_{3-5}$-Alkylen und

R Wasserstoff oder $C_{1-4}$-Alkyl,

oder eines seiner Salze,

gekennzeichnet durch

(A) Carb(alk)oxyalkylierung eines N-geschützten 2-Amino-7-hydroxytetralins der Formel II,

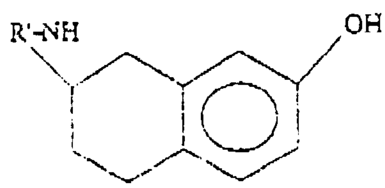

(II),

in der R' eine Aminoschutzgruppe bedeutet, die durch katalytische Hydrierung oder saure Hydrolyse unter milden Bedingungen abgespalten werden kann, entweder mit einer Verbindung der Formel IIIa,

$$Hal - Alk^1 - COOR \quad (IIIa)$$

in der bedeuten:

R dasselbe wie oben definiert,

Hal ein Atom Chlor, Brom oder Jod

und

$Alk^1$ eine Gruppe

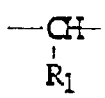 , 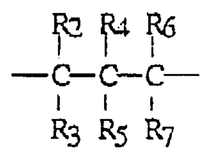 , 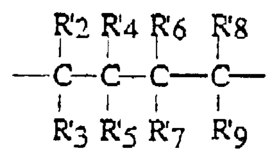

oder $-(CH_2)_5-$

wobei bedeuten:

R1 Ethyl, Propyl oder Butyl;

R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff,

in Gegenwart eines basischen Kondensationsmittels;

oder mit einer Verbindung der Formel IIIb,

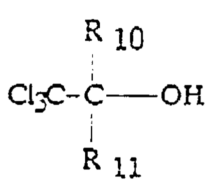

(IIIb),

worin bedeuten:

R10 und R11 unabhängig Methyl oder Ethyl, wobei R11 auch Propyl bedeuten kann, wenn R10 Methyl ist,

in Gegenwart einer starken Base;

sowie gegebenenfalls anschließende Umsetzung der so erhaltenen Verbindung mit Thionylchlorid in einem geeignet ausgewählten $C_{1-4}$-Alkanol;

oder mit einer Verbindung der Formel IIIc,

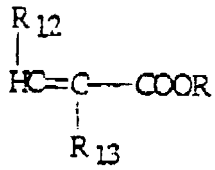

(IIIc),

worin bedeuten:

R dasselbe wie eben definiert

und

eine der Gruppen R12 und R13 Wasserstoff und die andere Methyl, Ethyl oder Propyl oder eine der Gruppen R12 und R13 Methyl und die andere Methyl oder Ethyl,

gegebenenfalls in Gegenwart katalytischer Mengen eines quaternären Ammoniumhydroxids, unter Erhalt eines N-geschützten 2-Amino-7-hydroxytetralins der Formel IV,

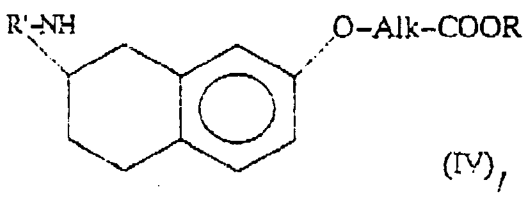

(IV),

in der Alk, R und R' die obige Bedeutung besitzen, und

(B) Abspaltung der N-Schutzgruppe durch katalytische Hydrierung oder saure Hydrolyse unter milden Bedingungen sowie, gegebenenfalls nach Verseifung der niederen Carbalkoxygruppe zu einer Carboxygruppe, Isolierung der Verbindung der Formel I in Form der freien Base oder eines Salzes und gegebenenfalls Umwandlung in eines ihrer Salze.

2. Verfahren nach Anspruch 1, dadurch gekennzeichnet, daß die N-Schutzgruppe R' unter Tertiärbutoxycarbonyl, Benzyloxycarbonyl, Benzyl, Benzhydryl und Trityl, die am Phenylkern nichtsubstituiert oder mit einer Methoxygruppe substituiert sein können, ausgewählt wird.

3. Verfahren nach Anspruch 1 oder 2, dadurch gekennzeichnet, daß Alk eine Gruppe

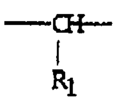 , 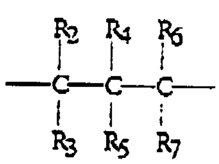 , 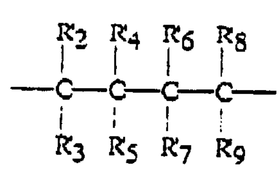 , $-(CH_2)_5-$ oder 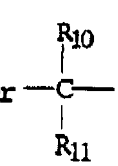

darstellt, wobei die Gruppen R1-R7, R10, R11 und R'2-R'9 die in Anspruch 1 definierte Bedeutung besitzen.

4. Verfahren nach Anspruch 3, gekennzeichnet durch Umsetzung einer Verbindung der Formel (II), in der R' Wasserstoff bedeutet,

* entweder mit einer Verbindung der Formel (IIIa), in der $Alk^1$ eine Gruppe

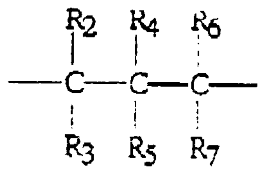 , 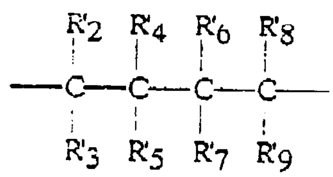

oder $-(CH_2)_5-$ darstellt, wobei R2-R7 und R'2 bis R'9 jeweils Wasserstoff und R Ethyl bedeuten,
* oder mit einer Verbindung der Formel (IIIb), in der R10 und R11 Methyl bedeuten,
zur Herstellung von:
2-Amino-7-(ethoxycarbonylpropan-3-yloxy)-tetralin,
2-Amino-7-(ethoxycarbonylbutan-4-yloxy)-tetralin,
2-Amino-7-(ethoxycarbonylpentan-5-yloxy)-tetralin und
2-Amino-7-(2-ethoxycarbonylpropan-2-yloxy)-tetralin
und deren Salzen.

5. Verfahren zur Herstellung eines Carboxyalkylethers eines N-geschützten 2-Amino-7-hydroxytetralins der Formel (IV),

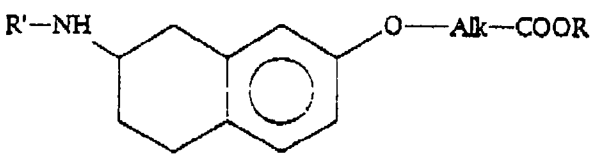

(IV),

in der R, Alk und R' die in Anspruch 1 definierte Bedeutung besitzen,
oder eines seiner Salze,
gekennzeichnet durch
Carb(alk)oxyalkylierung eines N-geschützten 2-Amino-7-hydroxytetralins der Formel II,

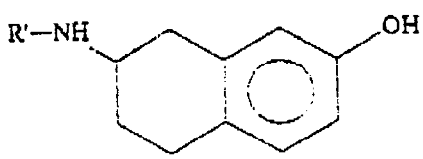

(II),

in der R' eine Aminoschutzgruppe bedeutet, die durch katalytische Hydrierung oder saure Hydrolyse unter milden Bedingungen abgespalten werden kann,
entweder mit einer Verbindung der Formel IIIa,

$$Hal - Alk^1 - COOR \quad (IIIa),$$

in der bedeuten:
R dasselbe wie oben definiert,
Hal ein Atom Chlor, Brom oder Jod
und
$Alk^1$ eine Gruppe

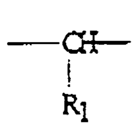 , 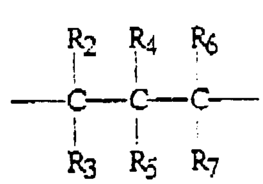 , 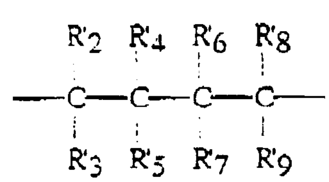

oder $-(CH_2)_5-$ wobei bedeuten:

R1 Ethyl, Propyl oder Butyl;

R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff, in Gegenwart eines basischen Kondensationsmittels;
oder mit einer Verbindung der Formel IIIb,

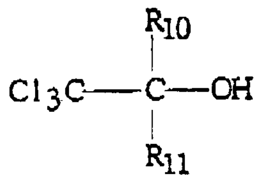

(III b),

worin bedeuten:
R10 und R11 unabhängig Methyl oder Ethyl, wobei R11 auch Propyl bedeuten kann, wenn R10 Methyl ist,
in Gegenwart einer starken Base;
sowie gegebenenfalls anschließende Umsetzung der so erhaltenen Verbindung mit Thionylchlorid in einem geeignet ausgewählten $C_{1-4}$-Alkanol;
oder mit einer Verbindung der Formel IIIc,

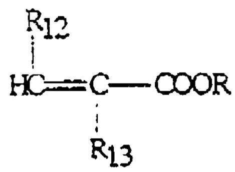

(IIIc),

worin bedeuten:
R dasselbe wie oben definiert
und
eine der Gruppen R12 und R13 Wasserstoff und die andere Methyl, Ethyl oder Propyl oder eine der Gruppen R12 und R13 Methyl und die andere Methyl oder Ethyl,
gegebenenfalls in Gegenwart katalytischer Mengen eines quaternären Ammoniumhydroxids
und gegebenenfalls Umwandlung in eines ihrer Salze.

6. Verfahren nach Anspruch 5, dadurch gekennzeichnet, daß R' die in Anspruch 2 definierte bedeutung besitzt.

7. Verfahren nach Anspruch 6, gekennzeichnet durch Umsetzung eine: verbindung der Formel (II) in der R' Benzyl bedeutet,
* entweder mit einer verbindung der Formel (IIIa), in der $Alk^1$ eine Gruppe

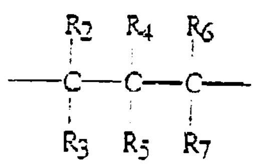

,

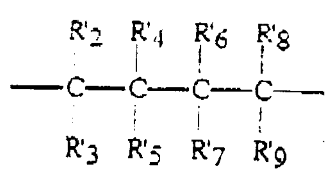

oder$-(CH_2)_5-$ darstellt, wobei R2-R7 und R'2-R'9 jeweils Wasserstoff und R Ethyl bedeuten,
* oder mit einer Vebindung der Formel (IIIc), in der R12 Methyl, R13 Wasserstoff und R Ethyl bedeuten, zur Herstellung der Verbindungen:
2-Benzylamino-7-(ethoxycarbonylpropan-3-yloxy)-tetralin,
2-Benzylamino-7-(ethoxycarbonylbutan-4-yloxy)-tetralin,
2-Benzylamino-7-(ethexycarbonylpentan-5-yloxy)-tetralin und
2-Benzylamino-7-(2-ethoxycarbonylpropan-2-yloxy)-tetralin
sowie deren Salzen.

8. Verfahren zur Herstellung eines Phenylethanolaminotetralins der formel XII,

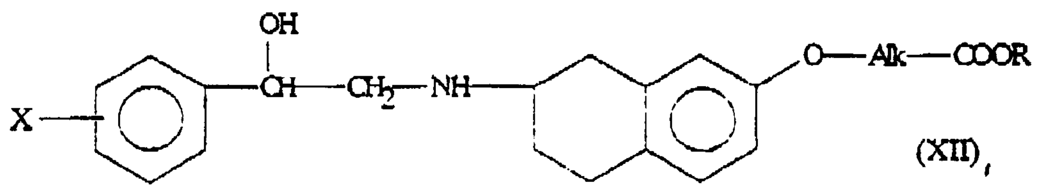

(XII),

in der bedeuten:
X Wasserstoff, Halogen, $C_{1-4}$-Alkyl oder Trifluormethyl,
R Wasserstoff oder $C_{1-4}$-Alkyl
und
Alk dasselbe wie in einem der Ansprüche 1 bis 3 definiert,
und seiner pharmazeutisch akzeptablen Salze,
gekennzeichnet durch
Umsetzung einer Verbindung der Formel I,

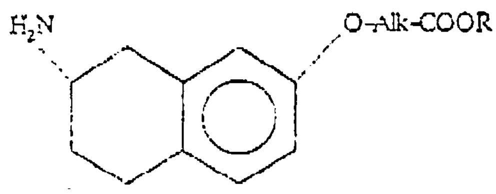

(I),

in der bedeuten:
Alk geradkettiges oder verzweigtes $C_{3-5}$-Alkylen und
R dasselbe wie oben definiert,

* entweder mit einem Styrolepoxid der Formel XIII,

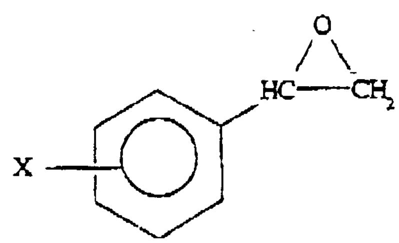

(XIII),

in der X die oben definierte Bedeutung besitzt,

* oder mit einem funktionellen Derivat einer Mandelsäure der Formel XIV,

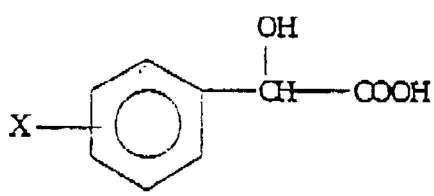

(XIV),

in der X die oben definierte Bedeutung besitzt,
und
Reduktion der Carbonsäureamidgruppe des so als Zwischenprodukt erhaltenen Mandelamids der Formel XV,

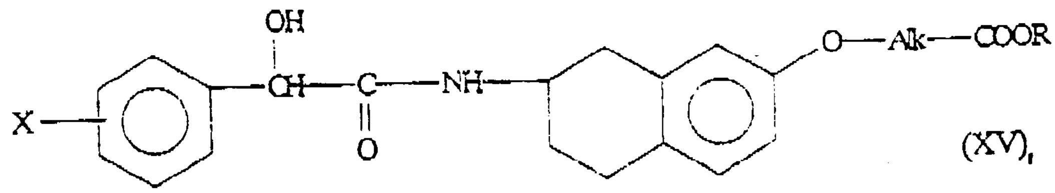

(XV),

in der X, Alk und R die oben definierte bedeutung besitzen, zu einer Methylengruppe und gegebenenfalls Umwandlung des so erhaltenen Produkts der Formel XII in eines seiner pharmazeutisch akzeptablen Salze.

9. Verfahren nach Anspruch 8, dadurch gekennzeichnet, daß Alk eine Gruppe

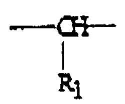 , 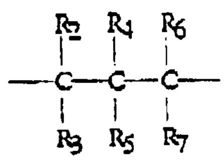 , 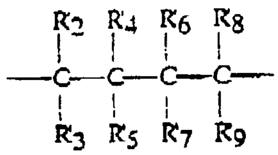 , $-(CH_2)_5-$ oder 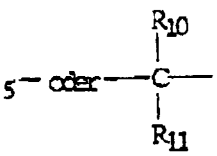

darstellt, wobei bedeuten:

R1 Ethyl, Propyl oder Butyl;

R2 bis R7 sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff;

R10 und R11 unabhängig Methyl oder Ethyl, wobei R11 ferner auch Propyl bedeuten kann, wenn R10 Methyl ist.

10. Verfahren nach Anspruch 9, dadurch gekennzeichnet, daß Alk eine Gruppe

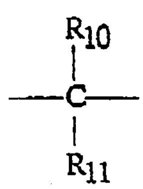

darstellt, in der R10 und R11 die in Anspruch 9 definierte Bedeutung besitzen.

11. Verfahren nach Anspruch 8 zur Herstellung von N-7-(ethoxycarbonylbutan-4-yloxy)-1,2,3,4-tetrahydronaphth-2-yl-2-hydroxy-2-(3-chlorphenyl)-ethanamin und seiner pharmazeutisch akzeptablen Salze, gekennzeichnet durch Umsetzung einer Verbindung der Formel (I), in der Alk eine Tetramethylengruppe und R eine Ethylgruppe bedeuten, entweder mit einer Verbindung der Formel (XIII), in der X eine 3-Chlor-Gruppe bedeutet, oder einer Verbindung der Formel (XIV), in der X eine 3-Chlor-Gruppe bedeutet.

12. Verfahren nach Anspruch 8 zur Herstellung von N-7-(ethoxycarbonylpropan-3-yloxy)-1,2,3,4-tetrahydronaphth-2-yl-2-hydroxy-2-(3-chlorphenyl)-ethanamin und seiner pharmazeutisch akzeptablen Salze, gekennzeichnet durch Umsetzung einer Verbindung der Formel (I), in der Alk eine Trimethylengruppe und R eine Ethylgruppe bedeuten, entweder mit einer Verbindung der Formel (XIII), in der X eine 3-Chlor-Gruppe bedeutet, oder mit einer Verbindung der Formel (XIV), in der X eine 3-Chlor-Gruppe bedeutet.

13. Verfahren nach Anspruch 8 zur Herstellung von N-7-(ethoxycarbonylpentan-5-yloxy)-1,2,3,4-tetrahydronaphth-2-yl-2-hydroxy-2-(3-chlorphenyl)-ethanamin und seiner pharmazeutisch akzeptablen Salze,

gekennzeichnet durch Umsetzung einer Verbindung der Formel (I), in der Alk eine Pentamethylengruppe und R eine Ethylgruppe bedeuten, entweder mit einer Verbindung der Formel (XIII), in der X eine 3-Chlor-Gruppe bedeutet, oder mit einer Verbindung der Formel (XIV), in der X eine 3-Chlor-Gruppe bedeutet.

**14.** Verfahren nach Anspruch 8 zur Herstellung von N-7-(2-ethoxycarbonylpropan-2-yloxy)-1,2,3,4-tetrahydronaphth-2-yl-2-hydroxy-2-(3-chlorphenyl)-ethanamin und seiner pharmazeutisch akzeptablen Salze, gekennzeichnet durch Umsetzung einer Verbindung der Formel (I), in der Alk eine Isopropylidengruppe und R eine Ethylgruppe bedeuten, entweder mit einer verbindung der Formel (XIII), in der X eine 3-Chlor-Gruppe bedeutet, oder mit einer Verbindung der Formel (XIV), in der X eine 3-Chlor-Gruppe bedeutet.

**Patentansprüche für folgenden Vertragsstaat : GR**

**1.** Carboxyalkylether des 2-Amino-7-hydroxytetralins der Formel I,

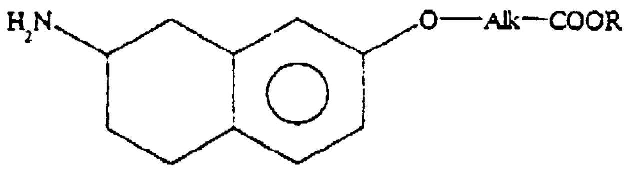

(I),

in der bedeuten:

Alk geradkettiges oder verzweigtes $C_{3-5}$-Alkylen und

R Wasserstoff oder $C_{1-4}$-Alkyl,

sowie eines seiner Salze.

**2.** Verbindungen nach Anspruch 1, in der Alk bedeutet:

(a) eine Gruppe

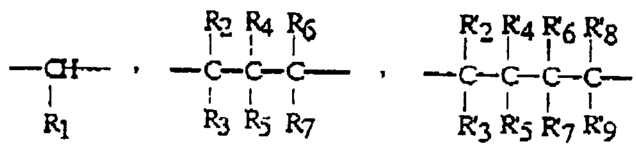

oder $-(CH_2)_5-$ wobei bedeuten:

R1 Ethyl, Propyl oder Butyl;

R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff;

(b) eine Gruppe

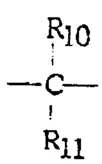

wobei bedeuten:

R10 und R11 unabhängig Methyl oder Ethyl, wobei R11 auch Propyl bedeuten kann, wenn R10 Methyl ist;

(c) eine Gruppe

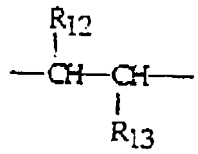

wobei bedeuten:
eine der Gruppen R12 und R13 Wasserstoff und die andere Methyl, Ethyl oder Propyl oder eine der Gruppen R12 und R13 Methyl und die andere Methyl oder Ethyl.

3. Verbindung nach Anspruch 2, wobei Alk eine Gruppe

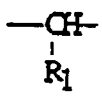 , 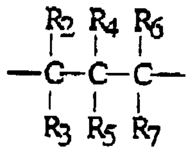 , 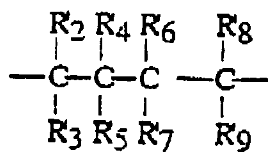 $-(CH_2)_5-$ od. 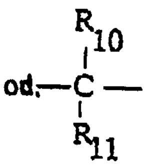

bedeutet, worin R1 - R7, R10, R11 und R'2 - R'9 die in Anspruch 2 definierte Bedeutung besitzen.

4. Verbindung nach Anspruch 3, die ausgewählt ist unter
2-Amino-7-(ethoxycarbonylpropan-3-yloxy)-tetralin,
2-Amino-7-(ethoxycarbonylbutan-4-yloxy)-tetralin,
2-Amino-7-(ethoxycarbonylpentan-5-yloxy)-tetralin, und
2-Amino-7-(2-ethoxycarbonylpropan-2-yloxy)-tetralin
sowie deren Salzen.

5. Verfahren zur Herstellung eines Carboxyalkylethers des 2-Amino-7-hydroxytetralin der Formel I,

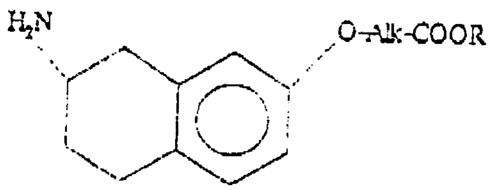

(I),

in der Alk und R die in Anspruch 1 definierte Bedeutung besitzen,
sowie eines seiner Salze,
gekennzeichnet durch
(A) Carb(alk)oxyalkylierung eines N-geschützten 2-Amino-7-hydroxytetralins der Formel II,

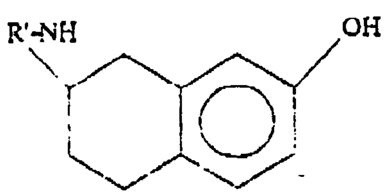

(II),

in der R' eine Aminoschutzgruppe bedeutet, die durch katalytische Hydrierung oder saure Hydrolyse unter milden Bedingungen abgespalten werden kann,
entweder mit einer Verbindung der Formel IIIa,

$$Hal - Alk^1 - COOR \quad (IIIa),$$

in der bedeuten:
R dasselbe wie oben definiert,
Hal ein Atom Chlor, Brom oder Jod

und
Alk[1] eine Gruppe

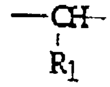 , 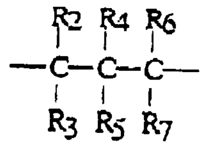 , 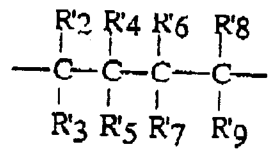 oder $-(CH_2)_5-$

wobei bedeuten:
R1 Ethyl, Propyl oder Butyl;
R2 bis R7 entweder sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;
R'2 bis R'9 sämtlich Wasserstoff oder eine der Gruppen R'2 bis R'9 Methyl und die übrigen Wasserstoff, in Gegenwart eines basischen Kondensationsmittels;
oder mit einer Verbindung der Formel IIIb,

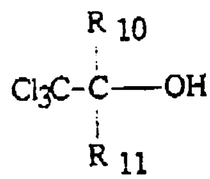 (IIIb),

worin bedeuten:
R10 und R11 unabhängig Methyl oder Ethyl, wobei R11 auch Propyl bedeuten kann, wenn R10 Methyl ist,
in Gegenwart einer starken Base;
sowie gegebenenfalls anschließende Umsetzung der so erhaltenen Verbindung mit Thionylchlorid in einem geeignet ausgewählten $C_{1-4}$-Alkanol;
oder mit einer Verbindung der Formel IIIc,

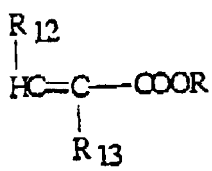 (IIIc),

worin bedeuten:
R dasselbe wie oben definiert
und
eine der Gruppen R12 und R13 Wasserstoff und die andere Methyl, Ethyl oder Propyl oder eine der Gruppen R12 und R13 Methyl und die andere Methyl oder Ethyl,
gegebenenfalls in Gegenwart katalytischer Mengen eines quaternären Ammoniumhydroxids,
unter Erhalt eines N-geschützten 2-Amino-7-hydroxytetralins der Formel IV,

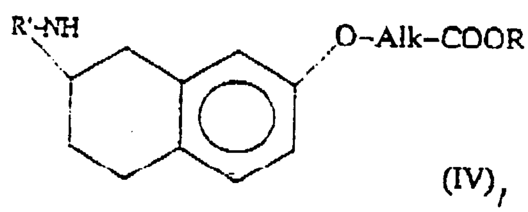 (IV),

in der Alk, R und R' die obige Bedeutung besitzen, und

(B) Abspaltung der N-Schutzgruppe durch katalytische Hydrierung oder saure Hydrolyse unter milden Bedingungen sowie, gegebenenfalls nach Verseifung der niederen Carbalkoxygruppe zu einer Carboxygruppe, Isolierung der Verbindung der Formel I in Form der freien Base oder eines Salzes und gegebenenfalls Umwandlung in eines ihrer Salze.

**6.** Verfahren nach Anspruch 5, dadurch gekennzeichnet, daß die N-Schutzgruppe R' unter Tertiärbutoxycarbonyl, Benzyloxycarbonyl, Benzyl, Benzhydryl und Trityl, die am Phenylkern nichtsubstituiert oder mit einer Methoxygruppe substituiert sein können, ausgewählt wird.

**7.** Carboxyalkylether eines N-geschützten 2-Amino-7-hydroxytetralins der Formel IV,

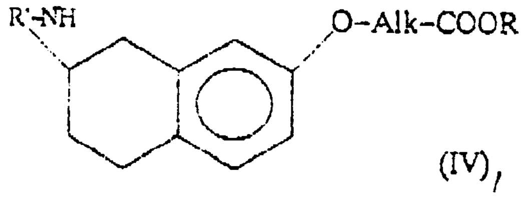

(IV),

in der R, Alk und R' die in Anspruch 5 definierte Bedeutung besitzen, oder eines seiner Salze.

**8.** Verbindung nach Anspruch 7, wobei R' die in Anspruch 6 definierte Bedeutung besitzt.

**9.** Verbindung nach Anspruch 8, die ausgewählt ist unter
2-Benzylamino-7-(ethoxycarbonylpropan-3-yloxy)-tetralin,
2-Benzylamino-7-(ethoxycarbonylbutan-4-yloxy)-tetralin,
2-Benzylamino-7-(ethoxycarbonylpentan-5-yloxy)-tetralin und
2-Benzylamine-7-(2-ethoxycarbonylpropan-2-yloxy)-tetralin
sowie deren Salzen.

**10.** Verfahren zur Herstellung eines Phenylethanolaminotetralins ser Formel XII,

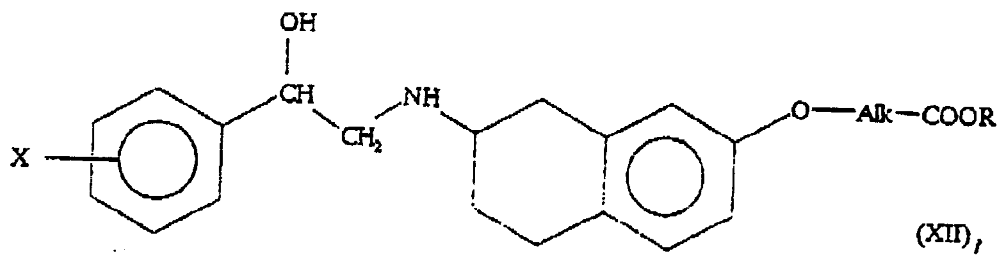

(XII),

in der bedeuten:
X Wasserstoff, Halogen, $C_{1-4}$-Alkyl oder Trifluormethyl,
R Wasserstoff oder $C_{1-4}$-Alkyl
und
Alk dasselbe wie in einem der Ansprüche 1 bis 3 definiert,
und seiner pharmazeutisch akzeptablen Salze,
gekennzeichnet durch
Umsetzung einer Verbindung der Formel I,

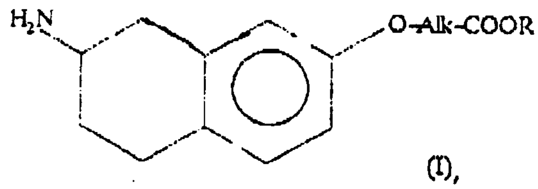

(I),

in der bedeuten:

Alk geradkettiges oder verzweigtes $C_{3-5}$-Alkylen und

R dasselbe wie oben definiert,

mit einem Styrolepoxid der Formel XIII,

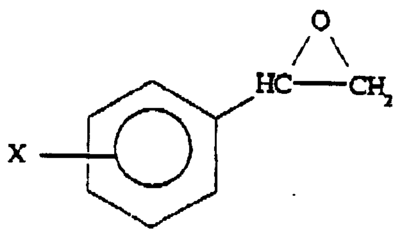

(XIII),

in der X die wie oben definierte Bedeutung besitzt,

oder Umsetzung mit einem funktionellen Derivat einer Mandelsäure der Formel XIV,

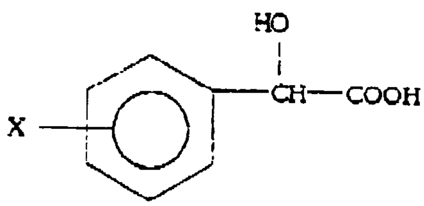

(XIV),

in der X die oben definierte Bedeutung besitzt, und

Reduktion der Carbonsäureamidgruppe des so als Zwischenprodukt erhaltenen Mandelamids der Formel XV,

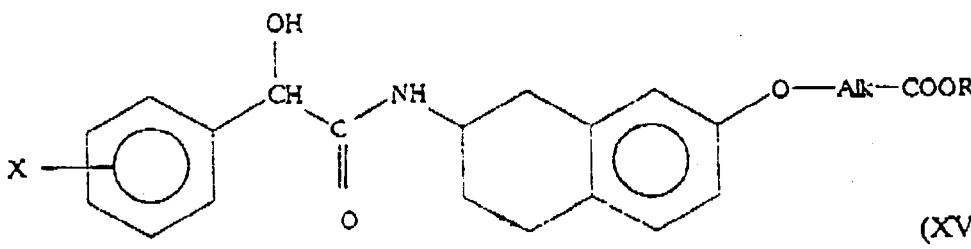

(XV),

in der X, Alk und R die oben definierte Bedeutung besitzen,

zu einer Methylengruppe

sowie gegebenenfalls Umwandlund der so erhaltenen Verbindung der Formel XII in eines ihrer pharmazeutisch akzeptablen Salze.

**11.** Verfahren nach Anspruch 10, dadurch gekennzeichnet, daß Alk eine Gruppe

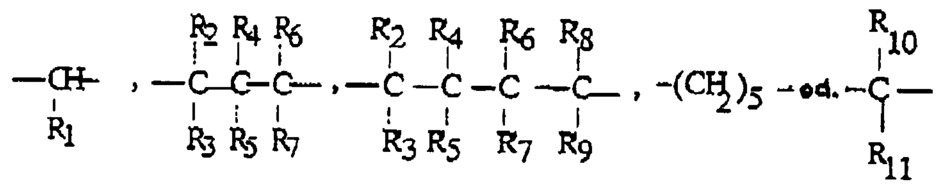

darstellt, wobei bedeuten:

R1 Ethyl, Propyl oder Butyl;

R2 bis R7 sämtlich Wasserstoff oder eine der Gruppen R2 bis R7 Methyl oder Ethyl und die übrigen Wasserstoff oder zwei der Gruppen R2 bis R7 Methyl und die übrigen Wasserstoff;

R'2 bis R'9 sämtlich Wasserstoff oder eine der Gruppen R'2 bis R'9 Methyl und die übrigen Wasserstoff;

R10 und R11 unabhängig Methyl oder Ethyl, wobei R11 auch Propyl sein kann, wenn R10 Methyl ist.

**12.** Verfahren nach Anspruch 11, dadurch gekennzeichnet, daß Alk eine Gruppe

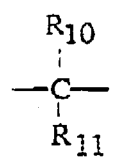

darstellt, wobei R10 und R11 die in Anspruch 11 definierte Bedeutung besitzen.

**13.** Verfahren nach Anspruch 10 zur Herstellung von N-[7-(ethoxycarbonylbutan-4-yloxy)-1,2,3,4-tetrahydronaphth-2-yl] -2-hydroxy-2-(3-chlorphenyl)-ethanamin und seiner pharmazeutisch akzeptablen Salze, gekennzeichnet durch Umsetzung einer Verbindung der Formel (I), in der Alk eine Tetramethylengruppe und R eine Ethylgruppe darstellen, entweder mit einer verbindung der Formel (XIII), in der X eine 3-Chlor-Gruppe bedeutet, oder einer Verbindung der Formel (XIV), in der X eine 3-Chlor-Gruppe bedeutet.

**14.** Verfahren nach Anspruch 10 zur Herstellung von N-[7-(ethoxycarbonylpropan-3-yloxy)-1,2,3,4-tetrahydronaphth-2-yl]-2-hydroxy-2-(3-chlorphenyl)-ethanamin und seiner pharmazeutisch akzeptablen Salze, gekennzeichnet durch Umsetzung einer Verbindung der Formel (I), in der Alk eine Trimethylengruppe und R eine Ethylgruppe darstellen, entweder mit einer Verbindung der Formel (XIII), in der X eine 3-Chlor-Gruppe bedeutet, oder mit einer Verbindung der Formel (XIV), in der X eine 3-Chlor-Gruppe bedeutet.

**15.** Verfahren nach Anspruch 10 zur Herstellung von N-[7-(ethoxycarbonylpentan-5-yloxy)-1,2,3,4-tetrahydronaphth-2-yl]-2-hydroxy-2-(3-chlorphenyl)-ethanamin und seiner pharmazeutisch akzeptablen Salze, gekennzeichnet durch Umsetzung einer Verbindung der Formel (I), in der Alk eine Pentamethylengruppe und R eine Ethylgruppe darstellen, entweder mit einer Verbindung der Formel (XIII), in der X eine 3-Chlor-Gruppe bedeutet, oder mit einer Verbindung der Formel (XIV), in der X eine 3-Chlor-Gruppe bedeutet.

**16.** Verfahren nach Anspruch 10 zur Herstellung von N-[7-(2-ethoxycarbonylpropan-2-yloxy)-1,2,3,4-tetrahydronaphth-2-yl]-2-hydroxy-2-(3-chlorphenyl)-ethanamin und seiner pharmazeutisch akzeptabler Salze, gekennzeichnet durch Umsetzung einer Verbindung der Formel (I), in der Alk eine Isopropylidengrupge und R eine Ethylgruppe darstellen, entweder mit einer Verbindung der Formel (XIII), in der X eine 3-Chlor-Gruppe bedeutet, oder mit einer Verbindung der Formel (XIV), in der X eine 3-Chlor-Gruppe bedeutet.

**17.** Verfahren zur Herstellung einer pharmazeutischen Zusammensetzung zur Behandlung gastrointestinaler Störungen, gekennzeichnet durch Mischen einer nach einem der Ansprüche 10 bis 16 hergestellten Verbindung mit einem pharmazeutisch akzeptablen Träger.